(19) Europäisches Patentamt
European Patent Office
Office européen des brevets

(11) EP 0 597 806 B1

(12) **EUROPÄISCHE PATENTSCHRIFT**

(45) Veröffentlichungstag und Bekanntmachung des Hinweises auf die Patenterteilung: **07.01.1998 Patentblatt 1998/02**

(51) Int. Cl.$^6$: **C07D 303/20**, C07C 217/52, C07C 215/12, C07C 43/196, C08G 59/20

(21) Anmeldenummer: **93810762.0**

(22) Anmeldetag: **02.11.1993**

(54) **Neue cyclohexylgruppenhaltige Glycidylether**
Novel cyclohexyl substituted glycidylethers
Nouveaux éthers glycidyliques substitués par un groupe cyclohéxyl

(84) Benannte Vertragsstaaten:
**CH DE ES FR GB IT LI NL**

(30) Priorität: **11.11.1992 CH 3488/92**

(43) Veröffentlichungstag der Anmeldung:
**18.05.1994 Patentblatt 1994/20**

(73) Patentinhaber:
**Ciba Specialty Chemicals Holding Inc.**
**4057 Basel (CH)**

(72) Erfinder:
- **Wolleb, Heinz, Dr.**
  **Ch-1723 Marly (CH)**
- **Kramer, Andreas, Dr.**
  **CH-3186 Düdingen (CH)**

(56) Entgegenhaltungen:

| | |
|---|---|
| EP-A- 0 467 290 | EP-A- 0 552 864 |
| FR-A- 1 539 419 | GB-A- 760 744 |
| GB-A- 1 053 193 | US-A- 3 244 731 |
| US-A- 3 327 016 | US-A- 4 088 614 |
| US-A- 4 767 883 | US-A- 5 128 491 |

- **CHEMICAL ABSTRACTS, vol. 93, no. 7, 18. August 1980, Columbus, Ohio, US; abstract no. 71525f, Seite 955 ; & JP-A-79 141 708 (OSAKA SODA CO., LTD.)**
- **CHEMICAL ABSTRACTS, vol. 107, no. 20, 16. November 1987, Columbus, Ohio, US; abstract no. 187292q, Seite 762 ; & CHEMICAL ABSTRACTS, 12TH COLLECTIVE INDEX, CHEMICAL SUBSTANCES Seite 36081CS 'mittlere Spalte, 19. Verbindung' & JP-A-62 136 658 (FUJI PHOTO FILM CO., LTD.)**
- **CHEMICAL ABSTRACTS, vol. 104, no. 6, 10. Februar 1986, Columbus, Ohio, US; abstract no. 43225k, Seite 625 ; & CHEMICAL ABSTRACTS, 11TH COLLECTIVE INDEX, CHEMICAL SUBSTANCES Seite 54761CS 'rechte Spalte, 1. Verbindung' & JP-A-60 131 289 (TOHO CHEMICAL INDUSTRY CO., LTD.)**
- **SYNTHESIS Nr. 3 , März 1978 , STUTTGART DE Seiten 223 - 225 D. LANDINI ET AL. 'Phase-transfer catalysts: Synthesis and catalytic activity of a tricyclohexyl[2.2.2]cryptand (perhydrotribenzohexaoxadiaza[8.8.8]eicosa ne'**
- **TETRAHEDRON LETTERS Bd. 29, Nr. 18 , 1988 , OXFORD GB Seiten 2215 - 2218 K. FURUTA ET AL. 'A direct synthesis of cyclic acetals from beta- or gamma-hydroxy ethers by means oc C-H activation'**
- **CHEMICAL ABSTRACTS, vol. 90, no. 3, 15. Januar 1979, Columbus, Ohio, US; abstract no. 22373j, 'Free-radical reaction of alicyclic alcohols with ethylene oxide' Seite 590 ; & DOKL. AKAD. NAUK AZ. SSR Bd. 34, Nr. 4 , 1978 Seiten 48 - 50**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.15.8/3.4

## Beschreibung

Die vorliegende Erfindung betrifft neue cyclohexylgruppenhaltige Glycidylether, Verfahren zu deren Herstellung und deren Verwendung z.B. in Epoxidharz-Formulierungen als Reaktivverdünner, Flexibilisatoren oder Adhäsionsverbesserer.

Cyclohexylgruppenhaltige Glycidylether, im allgemeinen als Epoxidharze bezeichnet, sind in grosser Zahl bekannt und können nach bekannten Arbeitsweisen hergestellt werden. Diese bestehen im Prinzip in einer katalysierten Kondensation einer geeigneten cyclohexylguppenhaltigen Hydroxyverbindung mit Epichlorhydrin oder seinen Derivaten in Gegenwart einer Lewissäure. Der Nachteil, der damit u.a. verbunden ist, ist der, dass in den nach dieser Art und Weise hergestellten Epoxidharzen ein hoher organisch gebundener Chloranteil (über etwa 1,0 Gew.%) vorhanden ist, der sich nachteilig (z.B. Korrosion, Vergilbung) bei den weiteren Verarbeitungsprodukten auswirkt (s. z.B. DE-A-2528022). Andererseits wird gemäss DE-A-3 629 632 vorgeschlagen, in Glycidylethern auf der Basis von aromatischen Bausteinen z.B. Bisphenol-A, Bisphenol-F, Phenol- und Kresol-Novolaken selektiv den aromatischen Ring zu hydrieren.

Im US-Patent 3,327,016 werden cyclohexylgruppenhaltige Glycidylether mit Chlormethygruppen beschrieben, die mit speziellen Aminhärtern zu transparenten Produkten ausgehärtet werden können.

Aromatische Epoxidharze mit Glycidyloxyalkylgruppen, die als elektrisches Isoliermaterial verwendet werden können, werden im US-Patent 3,244,731 offenbart.

Aufgabe der Erfindung war es nun, einen Weg zu finden, nach welchem einerseits wirtschaftlich cyclohexylgruppenhaltige Glycidylether mit einem tiefen Chlorgehalt hergestellt werden können und andereseits bei der Hydrierungsstute aromatische Ringe unter gleichzeitiger Ringöffnung des Oxiran-Ringes hydriert werden.

Die Lösung liegt in einem Verfahren, das keine Lewissäure verwendet. Es wurde gefunden, dass Alkohole, welche in β-Stellung eine Ether- bzw. Aminogruppe enthalten, mit einem Phasentransferverfanren gut mit Epihalohydrin umgesetzt werden können und so zu Harzen mit tiefem Chlorgehalt führen.

Die Erfindung betrifft demnach ein für diese Epoxidharze neues Verfahren, sowie neue Epoxidharze.

Die neuen Epoxidharze, bei welchen es sich um cyclohexylgruppenhaltige Glycidylether handelt, sind Verbindungen, die den folgenden Formeln I bis IV entsprechen:

(I)

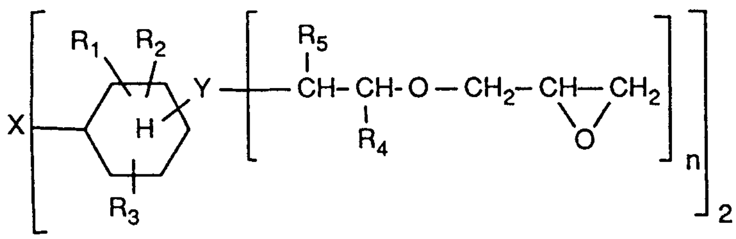

,

(II)

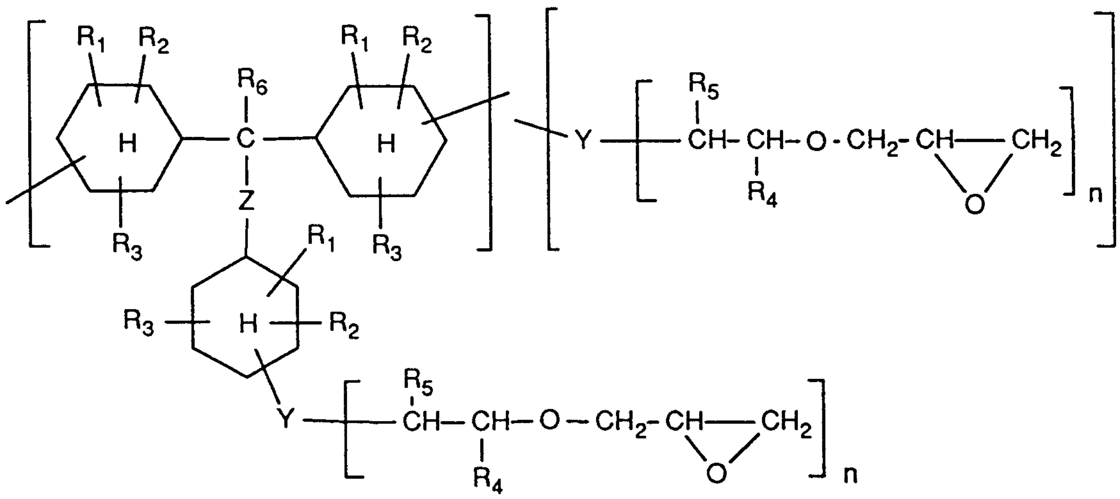

,

(III)

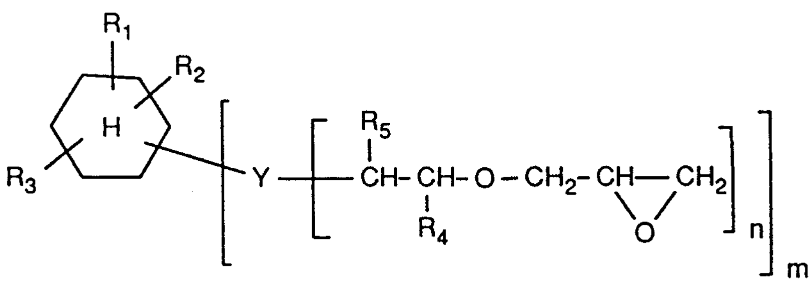

,

(IV)

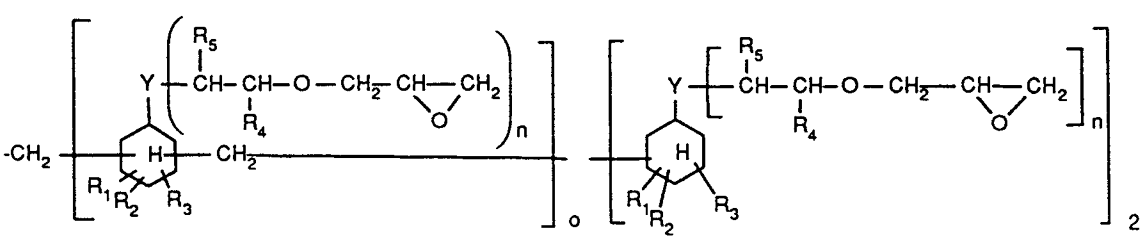

worin die einzelnen Symbole folgende Bedeutung haben:

$R_1$, $R_2$ und $R_3$ unabhängig voneinander Wasserstoff, einen gegebenenfalls substituierten $C_1$-$C_{10}$ Alkylrest oder einen hydrierten Arylrest;

$R_4$ und $R_5$ unabhängig voneinander Wasserstoff oder einen $C_1$-$C_{20}$ Alkylrest;

$R_6$ Wasserstoff, einen gegebenenfalls substituierten $C_1$-$C_{10}$ Alkylrest oder einen hydrierten Arylrest;
X ein Brückenglied der Formeln $-CH_2-$, $-C(CH_3)_2-$, $-C(CF_3)_2-$, -CO-, -O-, - S-, $-SO_2-$,

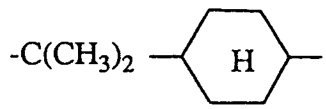 oder 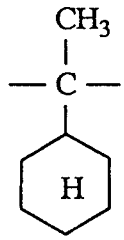 ;

Y = O (wenn n = 1) oder N (wenn n = 2);
m die Zahl 1, 2 oder 3;
n = 1 (wenn Y = O) oder 2 (wenn Y = N);
o die Zahl 1 bis 30; und
Z eine direkte Bindung oder ein Brückenglied der Formel $-CH_2-$ oder

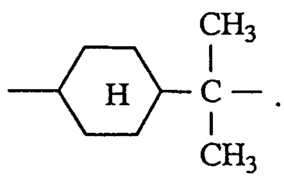

Bevorzugte Verbindungen sind symmetrisch und X und Y (in Formel I) stehen in p-Stellung zueinander, und die drei Y (in Formel II) stehen je in p-Stellung zu C (enthaltend $R_6$) beziehungsweise Z.

Des weiteren sind diejenigen Verbindungen der Formeln I bis IV bevorzugt, worin unabhängig voneinander

$R_1$, $R_2$ und $R_3$ Wasserstoff und/oder einen Alkylrest, vor allem einen $CH_3$-Rest; $R_4$ und $R_5$ Wasserstoff und/oder $CH_3$; $R_6$ Wasserstoff;
X ein Brückenglied der Formeln $-CH_2-$, $-C(CH_3)_2-$, $-C(CF_3)_2-$, -CO-, -O- oder - $SO_2$-;
Y O; n die Zahl 1;
Z eine direkte Bindung oder das Brückenglied $-CH_2-$;
m die Zahl 2 oder 3; und o die Zahl 1 bis 20 und insbesondere 1 bis 15, bedeuten.

Von Bedeutung sind vor allem die Verbindungen der Formeln I und IV.

Bedeuten $R_1$, $R_2$, $R_3$ und $R_6$ in den oben angegebenen Formeln I bis IV einen gegebenenfalls substituierten $C_1$-$C_{10}$ Alkylrest, so handelt es sich sowohl um geradkettige als auch um verzweigte Alkylreste, wie Methyl, Ethyl, Propyl, Isopropyl, Butyl, sek.-Butyl, tert.-Butyl, Pentyl, Isopentyl, Hexyl, Isohexyl und Octyl; all diese Alkylreste können ein- oder mehrmals durch z.B. Cyclohexyl substituiert sein.

Bedeutet $R_1$, $R_2$, $R_3$ und $R_6$ einen hydrierten Arylrest, so handelt es sich vor allem um einen hydrierten Benzol- oder Naphthalinrest.

$R_4$ und $R_5$ in der Bedeutung eines $C_1$-$C_{20}$ Alkylrestes stellen sowohl einen geradkettigen als auch verzweigten Alkylrest dar. Beispiele hierfür sind Methyl, Ethyl, Propyl, Isopropyl, Butyl, sek.-Butyl, Pentyl, Isopentyl, Hexyl, Octyl, n-Decyl, Dodecyl, Tetradecyl und Octadecyl.

In den bevorzugten Verbindungen der Formel I bedeuten

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
Y O,
n 1, und
X ein Brückenglied der Formeln $-CH_2-$, $-C(CH_3)_2-$, $-C(CF_3)_2-$, -CO-, -O- und $-SO_2-$.

In den bevorzugten Verbindungen der Formel II bedeuten

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
$R_6$ Wasserstoff,
Y O,
n 1, und
Z eine direkte Bindung oder ein Brückenglied $-CH_2-$.

In den bevorzugten Verbindungen der Formel III bedeuten

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
Y O,
n 1, und
m 2 oder 3, wobei, wenn m die Zahl 2 ist, die beiden Y-Reste vorzugsweise zueinander in 1,3-Stellung stehen und, wenn m 3 ist, in 1,3,5-Stellung.

In den bevorzugten Verbindungen der Formel IV bedeuten

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
Y O,
n 1, und
o die Zahl 1 bis 20, vor allem 1 bis 15.

All die Verbindungen der Formeln I bis IV, welche cycloaliphatische und Oxiran Elemente enthalten, sind bei 20°C in der Regel farblose bis gelbe Flüssigkeiten mit einer Viskosität von 10 bis 4000 mPa.s, insbesondere 100 bis 500 mPa.s, und sind vor allem durch einen sehr niederen Chlorgehalt von weniger als 0,4 Gew.% gekennzeichmet. Sie können über den Epoxidgehalt, Chlorgehalt, Molekulargewichtsverteilung (Mn, Mw) und die Viskosität charakterisiert werden.

Die Herstellung der Verbindungen der Formeln I bis IV kann beispielsweise nach zwei verschiedenen Verfahren a) und b) erfolgen:

Verfahren a)

Nach diesem Verfahren können die Verbindungen der Formeln I, II, III oder IV wie folgt hergestellt werden:

Die bekannten, und nach bekannter Art und Weise herstellbaren aromatischen Ausgangsverbindungen der Formeln Ia, IIa, IIIa oder IVa

(Ia) 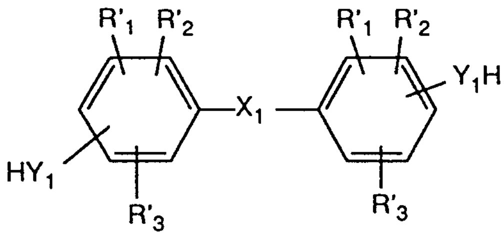 ,

(IIa) 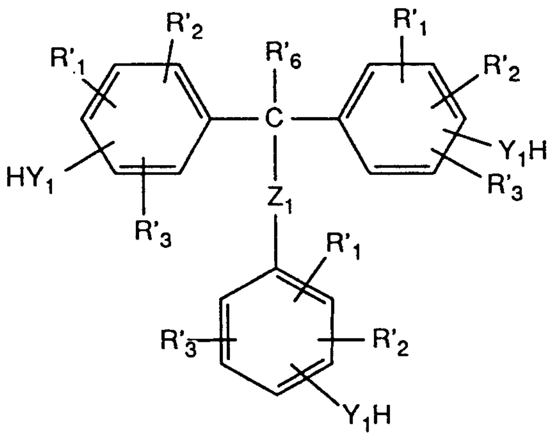 ,

(IIIa) 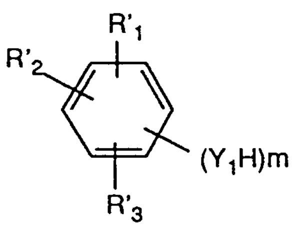 ,

(IVa) 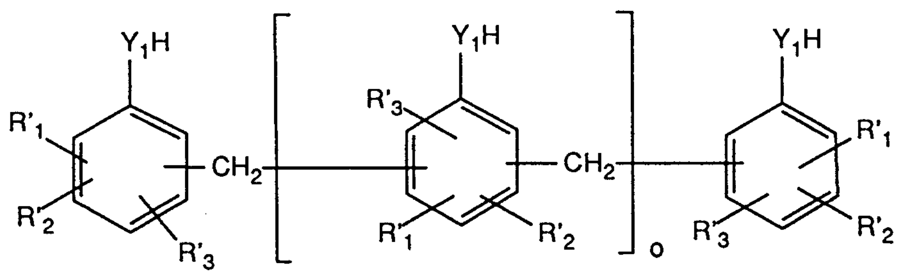 ,

werden in einem ersten Reaktionsschritt nach bekannten Verfahren zweckmässig mit etwa äquimolaren Mengen (∓ 5 %) Ethylenoxid oder dessen Derivaten der Formel

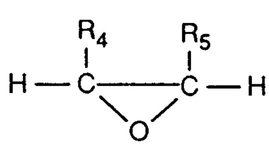 ,

worin $R_4$ und $R_5$ die unter den Formeln I bis IV angegebene Bedeutung haben, in Gegenwart einer Base in einem organischen Lösungsmittel bei einer Temperatur von etwa 50 bis 200°C und einem Druck von 1-10 bar umgesetzt. Die so erhaltenen Verbindungen der Formeln Ib, IIb, IIIb oder IVb

(Ib)

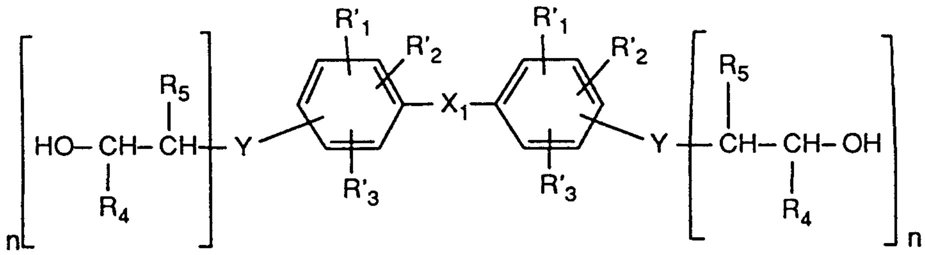

,

(IIb)

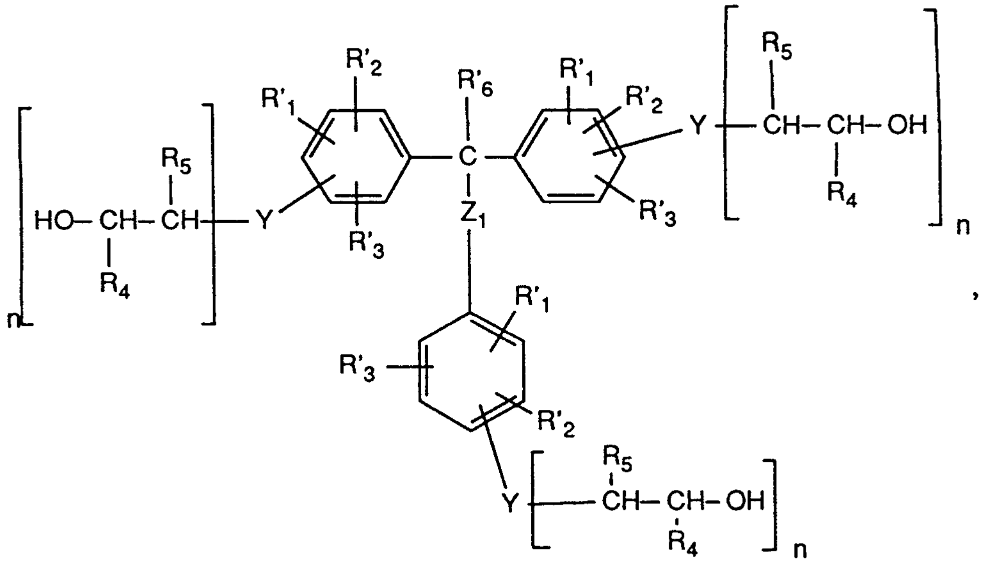

,

(IIIb)

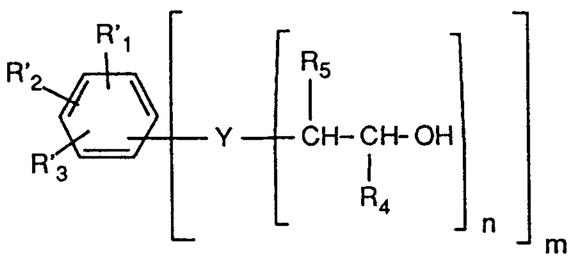

,

(IVb)

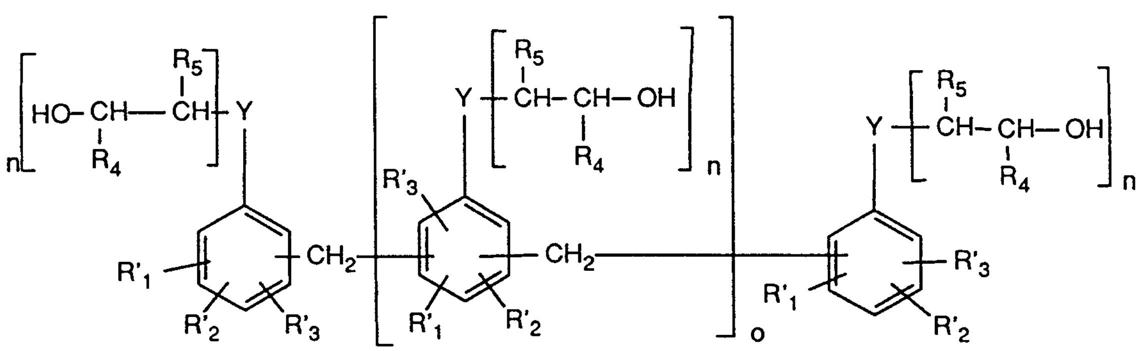

,

welche zum Teil ebenfalls bekannt sind, werden isoliert und in einem zweiten Reaktionsschritt katalytisch hydriert. Die Hydrierung erfolgt nach bekannten Verfahren mittels Wasserstoff und einem Katalysator zweckmässig in einem organischen Lösungsmittel bei einer Temperatur von etwa 50 bis 200°C und einem Druck von 50 bis 200 bar während etwa 2 bis 24 Stunden.

Die so erhaltenen hydrierten Verbindungen der Formeln Ic, IIc, IIIc oder IVc

(Ic) 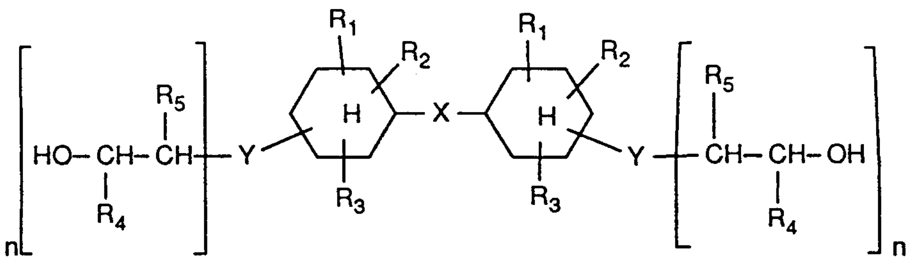 ,

(IIc)

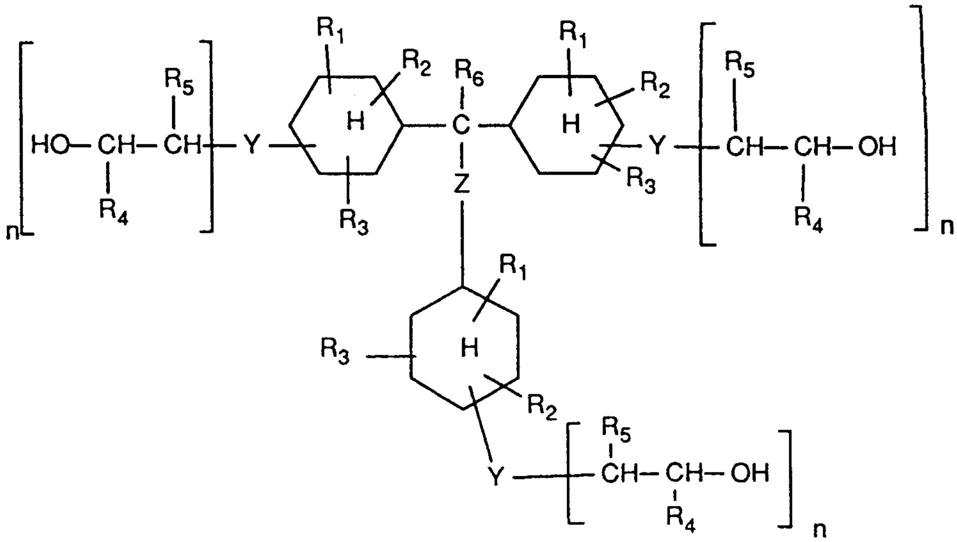 ,

(IIIc) 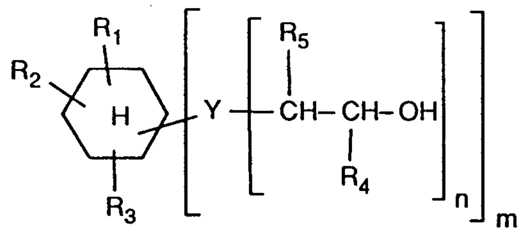 ,

(IVc)

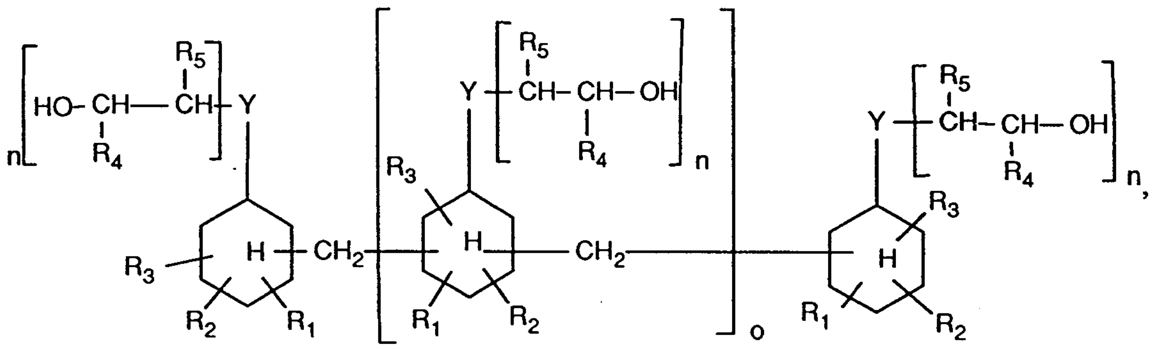 ,

werden isoliert und in einem dritten Reaktionsschritt nach bekannten Verfahren mit überschüssigem Epihalohydrin, z.B. Epichlorhydrin, in Gegenwart eines Phasentransferkatalysators bei einer Temperatur von 40 bis 90°C und einem Druck von 6665 bis 26660 Pa (50 bis 200 Torr) unter Zugabe einer Lauge zu den neuen Verbindungen der Formeln I, II, III bzw. IV umgesetzt.

In den obigen Ausgangs- und Zwischenprodukten haben die Symbole die gleiche Bedeutung der entsprechenden Symbole der erfindungsgemässen Verbindungen der Formeln I bis IV, mit Ausnahme vom Arylrest in $R'_1$, $R'_2$, $R'_3$, $R'_6$, $X_1$ und $Z_1$, wo der Arylrest jeweils einen nicht hydrierten Arylrest darstellt, und $Y_1$, welches O oder NH bedeutet.

Verfahren b)

Dieses Verfahren bietet eine Möglichkeit zur Herstellung von Verbindungen der Formeln I, II, III oder IV, worin $R_4$ $CH_3$ und $R_5$ H bedeuten.

Die bekannten, und nach bekannter Art und Weise herstellbaren Ausgangsverbindungen der Formeln $Ia_1$, $IIa_1$, $IIIa_1$ oder $IVa_1$

(Ia$_1$)

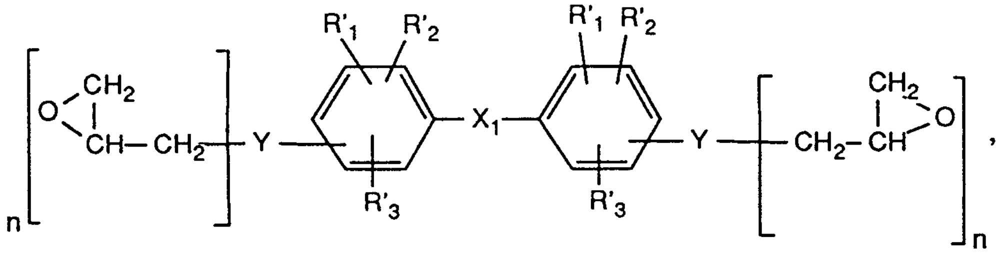

,

(IIa$_1$)

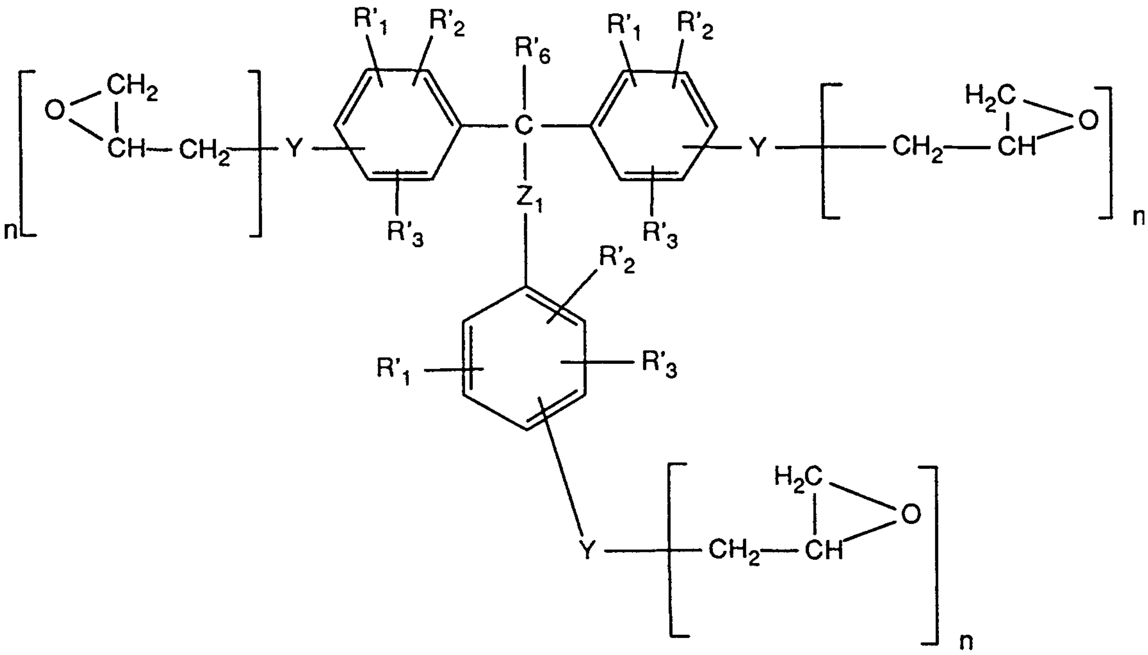

,

(IIIa$_1$)

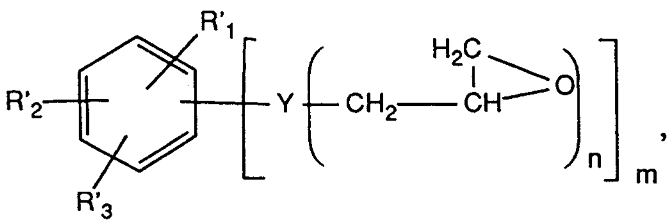

,

(IVa$_1$)

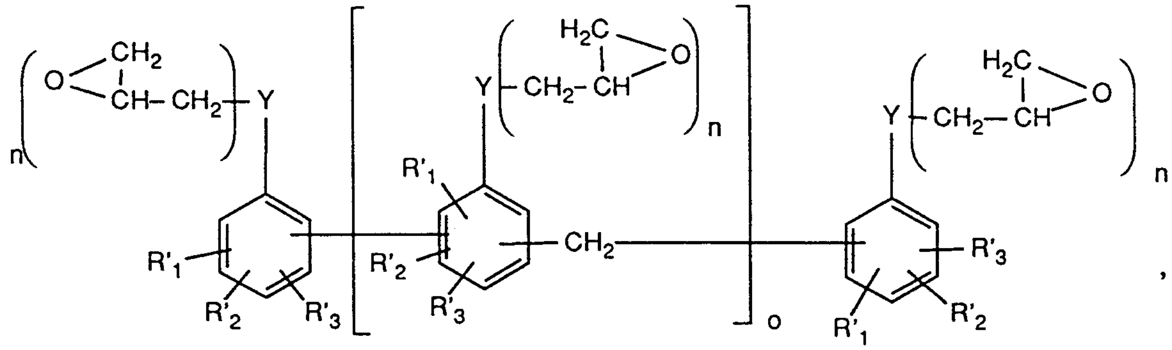

werden in einem ersten Reaktionsschritt katalytisch hydriert. Die Hydrierung erfolgt nach bekannten Verfahren mittels Wasserstoff und einem Katalysator zweckmässig in einem organischen Lösungsmittel bei einer Temperatur von etwa 50 bis 200°C und einem Druck von 50 bis 200 bar während etwa 2 bis 24 Stunden. Die so erhaltenen, hydrierten Verbindungen Ib$_1$, IIb$_1$, IIIb$_1$ und IVb$_1$

(Ib$_1$)

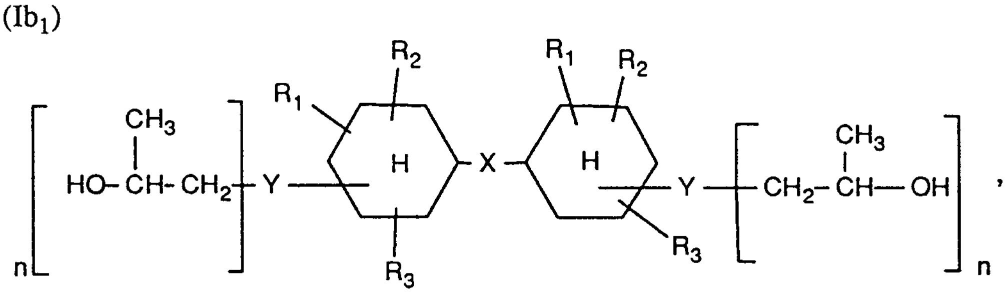

(IIb$_1$)

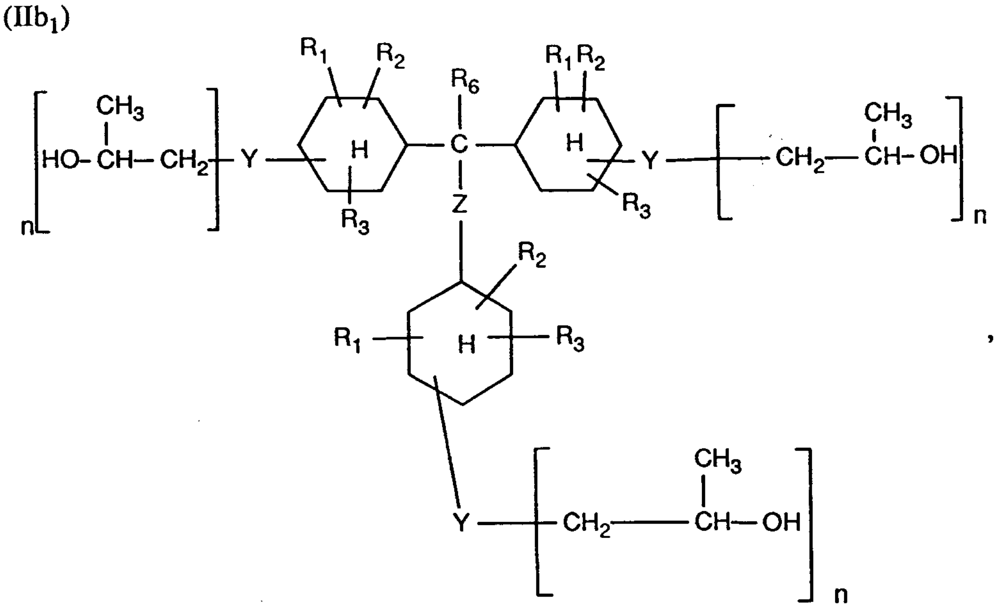

,

(IIIb$_1$)

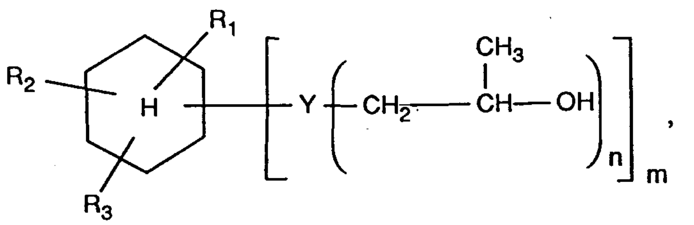

,

(IVb$_1$)

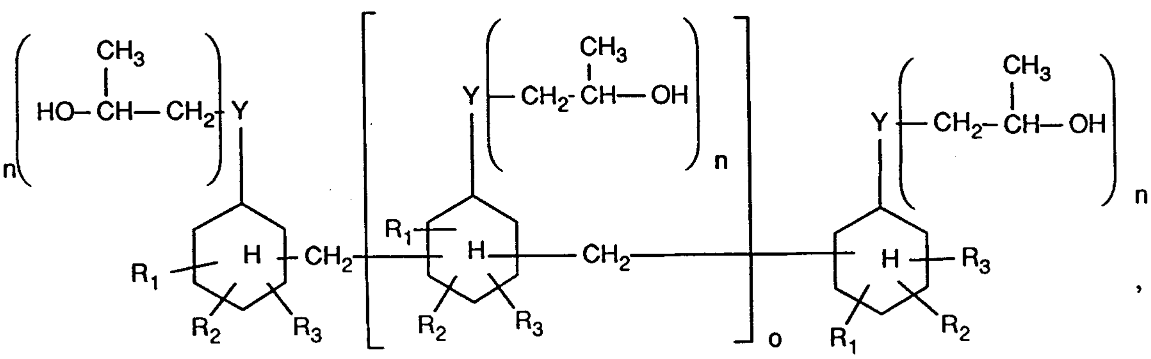

,

werden isoliert und in einem zweiten Reaktionsschritt nach bekannten Verfahren mit überschüssigem Epihalohydrin, z.B. Epichlorhydrin, in Gegenwart eines Phasentransferkatalysators bei einer Temperatur von 40 bis 90°C und einem Druck von 6665 bis 26660 Pa (50 bis 200 Torr) unter Zugabe einer Lauge zu den neuen Verbindungen der Formeln I, II, III beziehungsweise IV, worin $R_4$ $CH_3$ und $R_5$ H bedeuten, umgesetzt.

Als Epihalohydrin kommen z.B. Epibromhydrin und insbesondere Epichlorhydrin in Frage.

Bei den im Verfahren a) im ersten Reaktionsschritt eingesetzten Basen handelt es sich z.B. um sek. und tert. Amine, wie z.B. n-Ethyldiisopropylamin, Triethylamin, Tripropylamin, Tributylamin und Triisobutylamin.

Als geeignete organische Lösungsmittel kommen z.B. aromatische Kohlenwasserstoffe, wie Xylol oder Mesitylen, und vor allem aliphatische oder cycloaliphatische Ketone in Frage, wie Methylisobutylketon, Düsobutylketon, 2,4-Dimethyl-6-heptanon, 2-Heptanon, 3-Heptanon, Cyclopentanon, Methylcyclopentanon, Cyclohexanon und Methylcyclohexanon. Das bevorzugte Lösungsmittel ist Methylisobutylketon. Es können aber auch Gemische von verschiedenen Lösungsmitteln verwendet werden. Zweckmässig wird dieses Lösungsmittel in Mengen von 10 bis 90 Gew.%, bezogen

auf die gesamte Reaktionsmischung, eingesetzt.

Die Reaktionstemperatur liegt zweckmässig zwischen etwa 50 bis 200°C, vorzugsweise bei etwa 140 bis 160°C und insbesondere bei 150°C.

Der Reaktionsdruck bewegt sich zwischen 1 bis 10 bar und insbesondere zwischen 4 und 6 bar.

Die Hydrierung im Verfahren a) und b) wird mit Wasserstoff und einem Katalysator durchgeführt. Als Katalysatoren kommen z.B. in Frage: Raney Nickel, Ruthenium, Rhodium, Palladium und Platin. Diese Katalysatoren können auf inerten Trägermaterialien vorliegen, wie vor allem Aktivkohle, dann auf Sulfaten, Carbonaten oder Oxiden von Mg, Ba, Zn, Al, Fe, Cr und Zr, wie MgO, $ZiO_2$, $TiO_2$ und $\alpha$-$Al_2O_3$, oder auf Silicagel.

Als organische Lösungsmittel im Hydrier-Reaktionsschritt kommen z.B. in Frage:
Alkohole, wie Methanol und vor allem Ethanol oder Methylcellosolve, Ether, wie Tetrahydrofuran, und Ester, wie Essigsäureethylester.

Die Hydrierung wird zweckmässig bei einer Temperatur von 50 bis 200°C, vor allem 80 bis 180°C, bei einem Druck von 50 bis 200 bar, vor allem 100 bis 160 bar, und während einer Zeit von etwa 2 bis 24 Stunden, vor allem zwischen 10 und 15 Stunden, durchgeführt.

Die anschliessende Umsetzung mit Epihalohydrin im Verfahren a) und b) erfolgt vorzugsweise mit einem Ueberschuss von 2 bis 6 Mol pro OH-Gruppe der Verbindungen der Formel Ic, IIc, IIIc, IVc beziehungsweise $Ib_1$, $IIb_1$, $IIIb_1$ und $IVb_1$.

Als Phasentransferkatalysatoren kommen alle dem Fachmann bekannten Verbindungen in Frage, z.B. quartäre Ammoniumsalze und Phosphoniumsalze, wie z.B. die in dem US-Patent 4,465,722 erwähnten Tetraalkylammoniumhydroxide und -halogenide, sowie die in den US-Patenten 4,885,354, 4,973,648 und 5,006,626 beschriebenen Piperidinium-, Morpholinium- und Pyrrolidiniumsalze, wie beispielsweise:
Tetramethylammoniumchlorid, Tetrabutylammoniumbromid, N,N-Dimethylpyrrolidiniumchlorid, N-Ethyl-N-methylpyrrolidiniumiodid, N-Butyl-N-methylpyrrolidiniumbromid, N-Benzyl-N-methylpyrrolidiniumchlorid, N-Ethyl-N-methylpyrrolidiniumbromid, N-Butyl-N-methylmorpholiniumbromid, N-Butyl-N-methyl-morpholiniumiodid, N-Ethyl-N-hydroxyethylmorpholiniumiodid, N-Allyl-N-methylmorpholiniumbromid, N-Methyl-N-benzylpiperidiniumchlorid, N-Methyl-N-benzylpiperidiniumbromid, N,N-Dimethylpiperidiniumiodid, N-Methyl-N-ethylpiperidiniumacetat oder N-Methyl-N-ethylpiperidiniumiodid.
Vorzugsweise wird Tetramethylammoniumchlorid eingesetzt.

Diese Phasentransferkatalysatoren werden z.B. in einer Menge von etwa 0,01 bis 10 Mol %, vorzugsweise 1 bis 5 Mol % und insbesondere 2 bis 3 Mol %, bezogen auf das Ausgangsmaterial, eingesetzt.

Die Umsetzung mit dem Epihalohydrin, z.B. Epichlorhydrin, wird zweckmässig bei einer Temperatur von 40 bis 90°C, vor allem 50 bis 70°C und einem Druck von 6665 bis 26660 Pa (50 bis 200 Torr), vor allem 11997 bis 14666 Pa (90 bis 110 Torr) durchgeführt.

Der unter Rückfluss gehaltenen Mischung aus den Verbindungen Ic, IIc, IIIc, IVc bzw. $Ib_1$, $IIb_1$, $IIIb_1$ und $IVb_1$, dem Epihalohydrin und dem Phasentransferkatalysator wird vorzugsweise tropfenweise eine wässrige Alkalihydroxidlösung (Lauge), wie eine KOH- oder NaOH-Lösung, vor allem eine 50%ige wässrige NaOH-Lösung, innerhalb von 1 bis 5, vor allem innerhalb von 2 Stunden, zugetropft.

Die sowohl nach dem Verfahren a) als auch b) erhaltenen cyclohexylgruppenhaltigen Glycidylether der Formeln I bis IV werden anschliessend aufgearbeitet. Die Aufarbeitung erfolgt z.B. durch Abkühlung des Reaktionsgemisches auf Raumtemperatur, Filtration, Trocknung des Filterrückstandes (z.B. über $MgSO_4$) und Entfernung des überschüssigen Epihalohydrins aus dem Trocknungsprodukt.

Als Rückstand erhält man die erfindungsgemässen cyclohexylgruppenhaltigen Glycidylether der Formeln I bis IV mit einer Ausbeute über 70 % als farblose bis gelbe Flüssigkeiten oder Harze (bei 20°C), welche eine Viskosität von etwa 10 bis 4000 mPa.s, vor allem 100 bis 500 mPa.s aufweisen. Bedingt durch die Herstellungsverfahren sind diese niederviskosen Flüssigkeiten vor allem durch einen sehr niederen Chlorgehalt gekennzeichnet, der in über 95 % der Fälle kleiner als 0,4 Gew.%, vor allem kleiner als 0,3 Gew.% ist.

Es ist überraschend, dass die neuen cyclohexylgruppenhaltigen Glycidylether leicht nach dem Phasentransferverfahren hergestellt werden können infolge davon, dass die Ausgangsstoffe, $\beta$-Hydroxyether beziehungsweise $\beta$-Hydroxyamine, leicht glycidylisierbare OH-Gruppen enthalten; darüber hinaus ist es von Vorteil, dass keine Lewissäuren verwendet werden müssen.

Bei den bekannten Ausgangsverbindungen der Formeln Ia bis IVa handelt es sich z.B. um Dihydroxy- oder Diaminoverbindungen (Ia), um Verbindungen mit drei Hydroxy-bzw. Aminogruppen (IIa), um Verbindungen mit einer Hydroxygruppe bzw. Aminogruppe (IIIa) und um Verbindungen mit mehreren Hydroxy- bzw. Aminogruppen (IVa).

Beispiele für Dihydroxyverbindungen, die unter die obige Formel Ia fallen, sind Dihydroxyphenole, wie 4,4'-Dihydroxydiphenyldimethylmethan (Bisphenol A), 4,4'-Dihydroxydiphenylmethan, 3,3'-Dihydroxydiphenylmethan, 4,4'-Dihydroxydiphenyldifluormethylmethan, 2,2'-Dihydroxydiphenylketon 4,4'-Dihydroxydiphenylpropylphenylpropan oder 4,4'-Dihydroxydiphenylmethylphenylmethan, 4,4'-Diaminodiphenylmethan und 4,4'-Diaminodiphenyldimethylmethan.

Geeignete Derivate von Ethylenoxid, die sich mit diesen Dihydroxyverbindungen umsetzen lassen, sind beispielsweise ausser Ethylenoxid, Propylenoxid und Butylenoxid, Olefinoxide mit einer Kettenlänge von $C_1$-$C_{20}$; vorzugsweise verwendet man jedoch Ethylenoxid oder Propylenoxid.

Beispiele für Ausgangsverbindungen der Formel IIa sind z.B. 4,4',4"-Methylidyntrisphenol, 4,4',4"-Ethylidyn-trisphenol, 4,4'-[1(4(1-(4-hydroxyphenyl)-1-methylethyl)phenyl)ethyliden]-bisphenol und 4,4'-[4(1(4-hydroxyphenyl)-1-methylethyl)phenyl)methylen] -bisphenol.

Beispiele für die Ausgangsverbindungen der Formel IIIa sind z.B. Phenol, Anilin, Brenzkatechin, Resorcin, Phloroglucin und Pyrogallol.

Beispiele für die Ausgangsverbindungen der Formel IVa sind Novolake und Kresolnovolake.

Die erfindungsgemässen Verbindungen der Formeln I bis IV werden häufig in Epoxidharz-Formulierungen zur Modifikation bestimmter Eigenschaften eingesetzt, beispielsweise als Reaktivverdünner, Flexibilisatoren oder Adhäsionsverbesserer. Diese Formulierungen enthalten gegebenenfalls zusätzliche andere Epoxidharze, wie zum Beispiel Bisphenol A-Epoxidharze oder Epoxidnovolake, und übliche Härtungsmittel, wie zum Beispiel Amine, Carbonsäureanhydride, Phenole oder katalytische Härter. Die Formulierungen finden Anwendung in den verschiedensten Applikationsgebieten, beispielsweise als Lackharze, Tauchharze, Imprägnierharze, Klebstoffe, Dichtungsmittel, Umhüllungsmassen und Isoliermaterialien.

Die erfindungsgemässen Glycidylether weisen aufgrund ihrer aliphatischen Struktur ausgezeichnete Aussenbewitterungsbeständigkeit auf.

Die erfindungsgemässen Verbindungen können per se auch polymerisiert werden und als Lacke oder als Giessharze verwendet werden.

Die erfindungsgemässen Verbindungen ermöglichen die Herstellung z.B. von Produkten, die eine geringe Wasseraufnahme und eine gute Aussenbewitterungsbeständigkeit nebst guten allgemeinen Eigenschaften aufweisen.

Die folgenden Beispiele veranschaulichen die Erfindung, ohne sie darauf zu limitieren. Rh/C, Pd/C und Ru/C bedeutet Rhodium auf Kohle, bzw. Palladium auf Kohle, beziehungsweise ziehungsweise Ruthenium auf Kohle. Die Abkürzung TMAC bedeutet Tetramethylammoniumchlorid, und MIBK bedeutet Methylisobutylketon.

<u>Beispiel 1:</u> 320 g (1,01 Mol) 2,2'-bis[4-(2-hydroxyethoxy)phenyl]-propan (Herstellung z.B. nach JP-A 50105638) werden in einem Autoklaven in 2200 ml Ethanol in Gegenwart von 40 g Rh/C 5 % bei 80°C und 160 bar während 11 Stunden hydriert. Das Reaktionsgemisch wird filtriert und bei 70°C eingedampft. Man erhält 316 g (95 % der Theorie) eines leicht gelben Oeles, welches nach NMR keine aromatische Verbindung mehr enthält.

304 g (0,92 Mol) dieses Zwischenproduktes werden in 593 ml (7,56 Mol) Epichlorhydrin gelöst und mit 5,5 g einer 50%igen wässrigen Lösung von TMAC versetzt. Anschliessend werden unter Rückfluss bei 50-60°C Innentemperatur und 11997 Pa (90 Torr) innerhalb von 2 Stunden 163,5 g (2,04 Mol) einer 50%igen wässrigen NaOH-Lösung zugetropft, unter gleichzeitigem azeotropem Abdestillieren von Wasser. Danach wird noch 2 Stunden nachreagiert, abgekühlt und filtriert, das erhaltene Produkt wird über $MgSO_4$ getrocknet, und das Lösungsmittel wird eingedampft. Man erhält 398 g (98 % der Theorie) eines leicht gelben Harzes der nachstehenden Formel mit folgenden Eigenschaften:

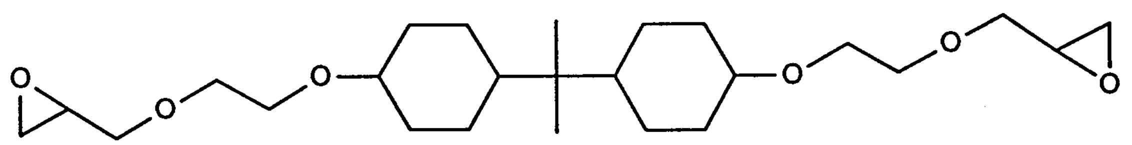

| | |
|---|---|
| Epoxidgehalt: | 4,06 Val/kg (89 % der Theorie) |
| Viskosität (25°C): | 240 mPa.s |
| Totaler Chlorgehalt: | 0,21 % |
| Hydrolisierbarer Chlorgehalt: | 283 ppm |

<u>Beispiel 2:</u> 400 g Bisphenol-F mit einem OH-Gehalt von 7,88 Val/kg, 2,6 g (0,02 Mol) N-Ethyldiisopropylamin und 200 ml MIBK werden in einem geeigneten Autoklaven mit 139,2 g (3,16 Mol) Ethylenoxid versetzt. Das Reaktionsgemisch wird anschliessend 5 Stunden bei 150°C gehalten, wobei der Druck bis auf 5,4 bar steigt. Anschliessend wird abgekühlt, und das Lösungsmittel wird am Rotavapor eingedampft. Man erhält 540 g (100 % der Theorie) eines braunen, sehr viskosen Oeles mit folgender Elementaranalyse: C = 71,33 %, H = 6,96 %.

536 g dieses Zwischenproduktes werden in 2800 ml Ethanol in Gegenwart von 50 g Rh/C 5 % bei 95° C und 160 bar während 14 Stunden hydriert. Das Reaktionsgemisch wird filtriert und bei 70° C eingedampft. Man erhält 547 g eines gelben, viskosen Oeles, welches nach NMR keine aromatische Verbindung mehr enthält.

150 g dieses Zwischenproduktes werden in 321 ml (4,1 Mol) Epichlorhydrin gelöst und mit 3,0 g einer 50%igen wässerigen TMAC-Lösung versetzt. Anschliessend werden unter Rückfluss bei 50-60°C Innentemperatur und 11997 Pa (90 Torr) innerhalb von 1 Stunde 88,0 g (1,10 Mol) einer 50%igen wässerigen NaOH-Lösung zugetropft, unter gleichzeitigem azeotropem Abdestillieren von Wasser. Danach wird noch 1,5 Stunden nachreagiert, abgekühlt und filtriert, das erhaltene Produkt wird über $MgSO_4$ getrocknet, und das Lösungsmittel wird eingedampft. Man erhält 200 g eines gelben Harzes der nachstehenden Formel mit folgenden Eigenschaften:

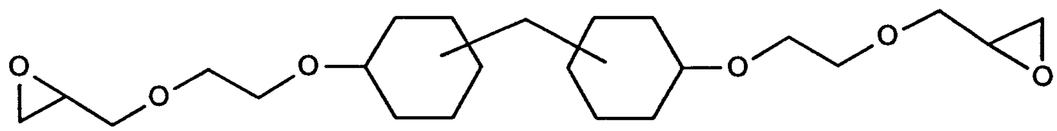

| | |
|---|---|
| Epoxidgehalt: | 4,05 Val/kg (84 % der Theorie) |
| Viskosität (25°C): | 360 mPa.s |
| Totaler Chlorgehalt: | 0,36 % |
| Hydrolisierbarer Chlorgehalt: | 820 ppm |

Beispiel 3: 200 g Härter HT9490 (Ciba-Geigy AG; Phenolnovolak) mit einem OH-Gehalt von 6,77 Val/kg, 1,7 g (0,013 Mol) N-Ethyldiisopropylamin und 200 ml MIBK werden in einem geeigneten Autoklaven mit 58,7 g (1,33 Mol) Ethylenoxid versetzt. Das Reaktionsgemisch wird anschliessend 5 Stunden bei 150°C gehalten, wobei der Druck bis auf 4,0 bar steigt. Anschliessend wird abgekühlt, und das Lösungsmittel wird am Rotavapor eingedampft. Man erhält 230 g (81 % der Theorie) eines orangen Feststoffes.

229 g dieses Zwischenproduktes werden in 2300 ml Ethanol in Gegenwart von 22 g Rh/C 5 % bei 160°C und 115 bar während 12 Stunden hydriert. Das Reaktionsgemisch wird filtriert und bei 70°C eingedampft. Man erhält 224 g eines gelben Feststoffes, welches nach NMR keine aromatische Verbindung mehr enthält.

220 g dieses Zwischenproduktes werden in 532 g (5,74 Mol) Epichlorhydrin gelöst und mit 8,4 g einer 50%igen wässerigen TMAC-Lösung versetzt. Anschliessend werden unter Rückfluss bei 50-60°C Innentemperatur und 11997 Pa (90 Torr) innerhalb von 1 Stunde 88,0 g (1,10 Mol) einer 50%igen wässerigen NaOH-Lösung zugetropft, unter gleichzeitigem azeotropem Abdestillieren von Wasser. Danach wird noch 1,0 Stunde nachreagiert, dann abgekühlt, filtriert, über $MgSO_4$ getrocknet, und das Lösungsmittel wird eingedampft. Man erhält 271 g eines gelben Harzes der nachstehenden Formel mit folgenden Eigenschaften:

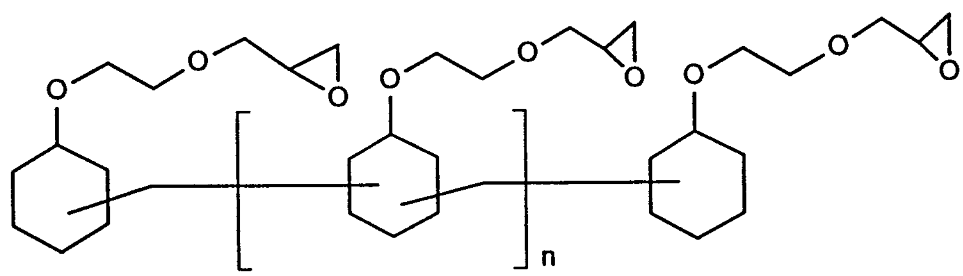

| | |
|---|---|
| Epoxidgehalt: | 4,28 Val/kg (90% der Theorie) |
| Viskosität (25°C): | 3200 mPa.s |
| Totaler Chlorgehalt: | 0,20 % |
| Hydrolisierbarer Chlorgehalt: | 380 ppm |

Beispiel 4: 450 g (1,32 Mol) Araldit MY 790 (Ciba-Geigy AG; Bisphenol-A-diglycidylether) werden in 4300 ml Methylcellosolve in Gegenwart von 22 g Ru/C 10 % bei 100 bar und 180°C hydriert. Anschliessend wird das Reaktionsgemisch abgekühlt, filtriert, und das Lösungsmittel wird am Rotavapor abdestilliert. Der Rückstand wird in 1 l Essigester gelöst, 3- mal mit 100 ml Wasser gewaschen, über $MgSO_4$ getrocknet, filtriert und am Rotavapor eingedampft. Man erhält 470 g (100 % der Theorie) eines farblosen Oeles, welches nach NMR keine aromatische Verbindung mehr enthält und in dem durch Titration keine Epoxidgruppen mehr nachweisbar sind.

285,2 g (0,8 Mol) dieses Zwischenproduktes werden in 376 ml (4,75 Mol) Epichlorhydrin gelöst und mit 4,64 g einer 50%igen wässerigen TMAC-Lösung versetzt. Anschliessend werden unter Rückfluss bei 60-70°C Innentemperatur und 14666 Pa (110 Torr) innerhalb von 2 Stunden 140,8 g (1,75 Mol) einer 50%igen wässrigen NaOH-Lösung zugetropft, unter gleichzeitigem azeotropem Abdestillieren von Wasser. Danach wird noch 3,5 Stunden nachreagiert, abgekühlt, filtriert, über $MgSO_4$ getrocknet, und das Lösungsmittel wird eingedampft. Man erhält 325 g (87 % der Theorie) eines

gelben Harzes der nachstehenden Formel mit folgenden Eigenschaften:

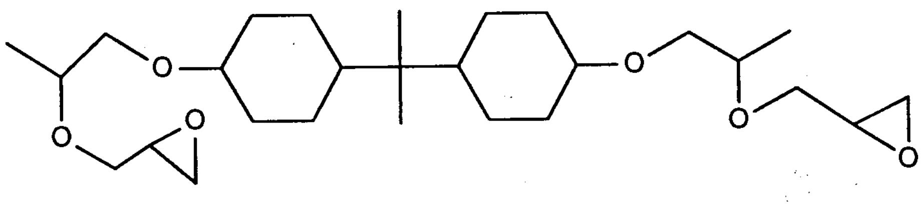

| | |
|---|---|
| Epoxidgehalt: | 3,29 Val/kg (77 % der Theorie) |
| Viskosität (25°C): | 440 mPa.s |
| Totaler Chlorgehalt: | 0,24 % |
| Hydrolisierbarer Chlorgehalt: | 198 ppm |

Beispiel 5: 213 g Araldit PY 307 (Ciba-Geigy AG; Bisphenol-F-diglycidylether) werden in 2130 ml Methylcellosolve in Gegenwart von 20 g Ru/C 10 % bei 110 bar und 180°C hydriert. Anschliessend wird das Reaktionsgemisch abgekühlt und filtriert, und das Lösungsmittel wird am Rotavapor abdestilliert. Man erhält 225 g eines leicht gelben Oeles, welches nach NMR keine aromatische Verbindung mehr enthält und in dem durch Titration keine Epoxidgruppen mehr nachweisbar sind.

224 g dieses Zwischenproduktes werden in 520 g (5,60 Mol) Epichlorhydrin gelöst und mit 4,10 g einer 50%igen wässerigen TMAC-Lösung versetzt. Anschliessend werden unter Rückfluss bei 50-60°C Innentemperatur und 11997 Pa (90 Torr) innerhalb von 1 Stunde 120,0 g (150 Mol) einer 50%igen, wässerigen NaOH-Lösung zugetropft, unter gleichzeitigem azeotropem Abdestillieren von Wasser. Danach wird noch 2 Stunden nachreagiert, abgekühlt, filtriert, über $MgSO_4$ getrocknet, und das Lösungsmittel wird eingedampft. Man erhält 297 g eines gelben Harzes mit folgenden Eigenschaften:

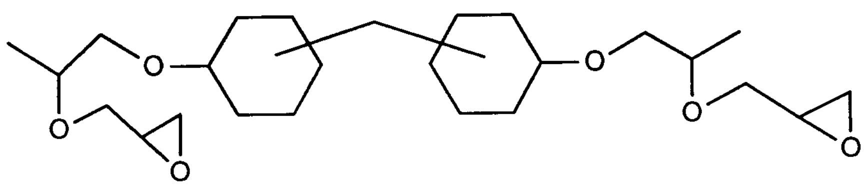

| | |
|---|---|
| Epoxidgehalt: | 3,35 Val/kg (74 % der Theorie) |
| Viskosität (25°C): | 320 mPa.s |
| Totaler Chlorgehalt: | 0,34 % |
| Hydrolisierbarer Chlorgehalt: | 830 ppm |

Beispiel 6: 267 g Araldit EPN 1139 (Ciba-Geigy AG; Epoxy-Kresolnovolak) werden in 2700 ml Methylcellosolve in Gegenwart von 26 g Pd/C 5 % bei 115 bar und 160°C hydriert, bis kein Wasserstoff mehr aufgenommen wird (30 Stunden). Anschliessend wird das Reaktionsgemisch abgekühlt und filtriert, und das Lösungsmittel wird am Rotavapor abdestilliert. Man erhält 297 g eines leicht gelben Oeles, welches nach NMR keine aromatische Verbindung mehr enthält und in dem durch Titration keine Epoxidgruppen mehr nachweisbar sind.

250 g dieses Zwischenproduktes werden in 560 g (6 Mol) Epichlorhydrin gelöst und mit 4,4 g einer 50%igen wässerigen TMAC-Lösung versetzt. Anschliessend werden unter Rückfluss bei 50-60°C Innentemperatur und 11997 Pa (90 Torr) innerhalb 1 Stunde 120,0 g (1,50 Mol) einer 50%igen, wässerigen NaOH-Lösung zugetropft, unter gleichzeitigem azeotropem Abdestillieren von Wasser. Danach wird noch 2 Stunden nachreagiert, abgekühlt, filtriert und über $MgSO_4$ getrocknet, und das Lösungsmittel wird eingedampft. Man erhält 236 g eines gelben Harzes der nachstehenden Formel mit folgenden Eigenschaften:

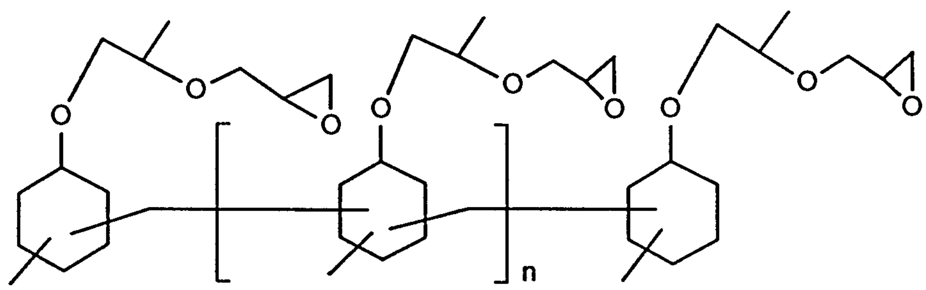

| | |
|---|---|
| Epoxidgehalt: | 3,41 Val/kg (76 % der Theorie) |
| Viskosität (25°C): | 254 mPa.s |
| Totaler Chlorgehalt: | 0,66 % |
| Hydrolisierbarer Chlorgehalt: | 240 ppm |
| $M_n$: | 503 |
| $M_w$: | 796 |
| $M_w/M_n$: | 1,58 |

Beispiel 7: 15 g Araldit MY 0510 (Ciba-Geigy AG; 4-Aminophenyltriglycid) werden in 150 ml Methylcellosolve in Gegenwart von 1,5 g Ru/C bei 110°C und 100 bar hydriert, bis kein Wasserstoff mehr aufgenommen wird (20 Stunden). Anschliessend wird das Reaktionsgemisch abgekühlt und filtriert, und das Lösungsmittel wird am Rotavapor abdestilliert. Man erhält 15 g eines braunen Oeles, welches nach NMR keine aromatische Verbindung mehr enthält und nur noch 30% des theoretischen Epoxidgehaltes aufweist. 15 g dieses Zwischenproduktes werden in 58 g (0,62 Mol) Epichlorhydrin gelöst und mit 0,46 g einer 50%igen wässerigen TMAC-Lösung versetzt. Anschliessend werden unter Rückfluss bei 50-60°C Innentemperatur und 11997 Pa (90 Torr) innerhalb von 1 Stunde 12,8 g (0,16 Mol) einer 50%igen, wässrigen NaOH-Lösung zugetropft, unter gleichzeitigem azeotropen Abdestillieren von Wasser. Danach wird noch 1 Stunde nachreagiert, abgekühlt, filtriert und über $MgSO_4$ getrocknet, und das Lösungsmittel wird eingedampft. Man erhält 15,32 g eines gelben Harzes der nachstehenden Formel mit folgenden Eigenschaften:

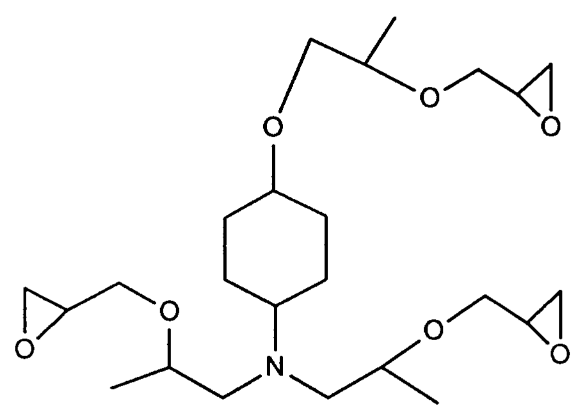

| | |
|---|---|
| Epoxidgehalt: | 6,39 Val/kg (97 % der Theorie) |
| Viskosität (25°C): | 150 mPa.s |
| Totaler Chlorgehalt: | 0,31 % |
| Hydrolisierbarer Chlorgehalt: | 780 ppm |

Beispiel 8: 121,8 g des Härters HT 907 (Ciba-Geigy AG; Hexahydrophthalsäureanhydrid) und 0,7 g des Beschleunigers DY062 (Ciba-Geigy AG; Benzyldimethylamin) werden vorgelegt und bei 60°C geschmolzen und unter Rühren gemischt. Dann werden 112,0 g LMB5132 (Ciba-Geigy AG; Hexahydrophthalsäurediglycidylester) und 28,0 g des nach Beispiel 5 hergestellten gelben Harzes zugegeben und bei 60°C homogenisiert. Anschliessend werden 393,4 g Quarzmehl W12EST (Quarzwerke Frechen), fein verstaubt, portionenweise eingestreut und 10 Min. unter Rühren bei 80°C

eingemischt. Danach lässt man die Gesamtmischung unter Vakuum (1 mbar) und Rühren laufen (ca. 7 Min.), bis die Mischung luftfrei ist. Anschliessend wird diese Mischung in 100°C vorgewärmte Platten gegossen und für 2h bei 100°C und 16h bei 140°C gehärtet.

Man erhält Platten mit den folgenden Eigenschaften:

| | |
|---|---|
| Biegefestigkeit: | 149 $N/mm^2$ |
| Randfaserdehnung: | 1,79 $N/mm^2$ |
| Zugfestigkeit (23°C): | 91 $N/mm^2$ |
| Double Torsion $G_{-IC}$: | 486 $J/m^2$ |
| Double Torsion $K_{-IC}$: | 2,3 $MPa.m^{1/2}$ |
| $T_g$ (DSC): | 99°C |

Beispiel 9: 100 g des Harzgemisches aus 60 g MY-721 (Ciba-Geigy AG: 4,4'-Diaminodiphenylmethan-tetraglycid) und 40 g des nach Beispiel 5 hergestellten gelben Harzes wurden auf 140°C erhitzt. Dann wurden unter Rühren 37 g des Härters HT 976 (Ciba-Geigy AG; 4,4'-Diaminodiphenylsulfon) zugegeben. Nachdem sich der Härter vollständig gelöst hatte, wurde die Mischung für 5 Min. bei 10 mm Hg entgast. Anschliessend wurde die Mischung in 180°C heisse Aluminiumformen (4 mm) gegossen und für 4h bei 180°C gehärtet.

Man erhält Platten mit den folgenden Eigenschaften:

| | |
|---|---|
| $Tg_0$ (TMA): | 224°C |
| Tg (TMA): | 215°C |
| E-Modulus: | 4060 ± 65 MPa |
| Strength: | 109 ± 22 MPa |
| Elongation: | 2,7 ± 0,6 % |

Die Formulierung kann als Matrixharz zusammen mit Fasern (Kohle, Glas, Aramid, Polyester etc.) zu hochwertigen Faserverbundstoffen mit hoher Flexibilität und Wärmebeständigkeit verwendet werden.

**Patentansprüche**

**1.** Cyclohexylgruppenhaltige Glycidylether der Formeln I, II, III und IV:

(I)

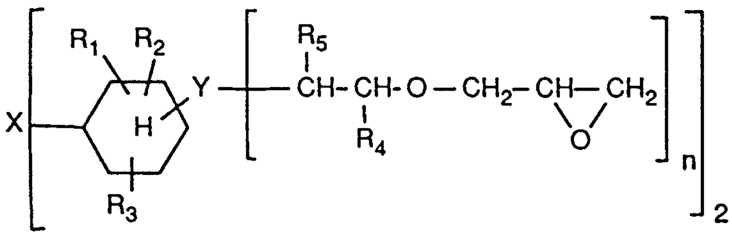

,

(II)

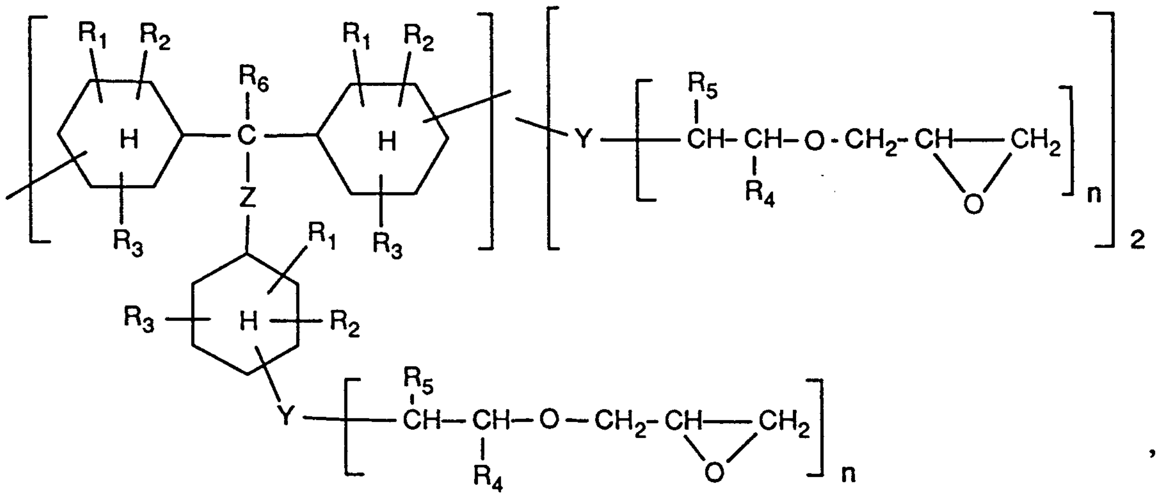

,

(III)

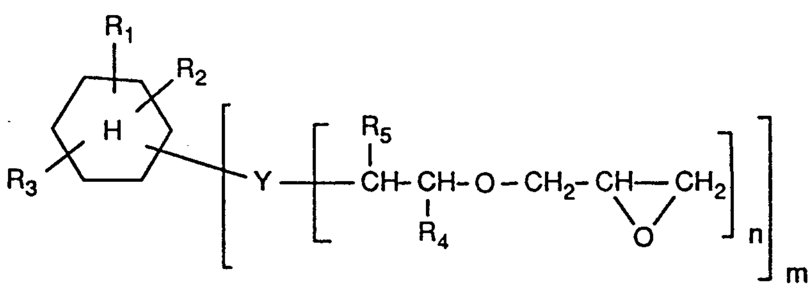

,

(IV)

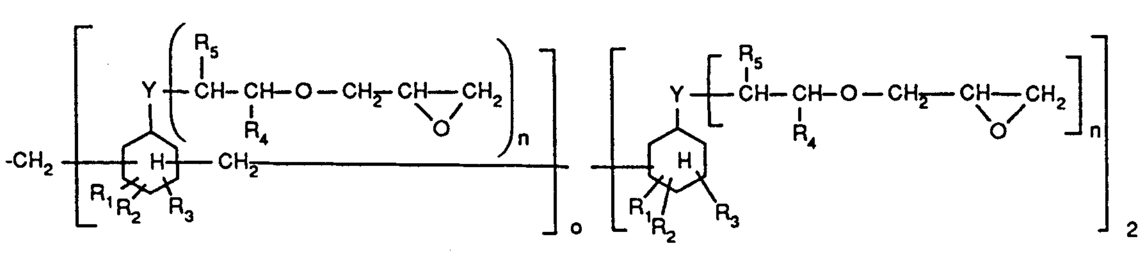

worin die einzelnen Symbole folgende Bedeutung haben:

$R_1$, $R_2$ und $R_3$ unabhängig voneinander Wasserstoff, einen gegebenenfalls substituierten $C_1$-$C_{10}$ Alkylrest

oder einen hydrierten Arylrest;

$R_4$ und $R_5$ unabhängig voneinander Wasserstoff oder einen $C_1$-$C_{20}$ Alkylrest;

$R_6$ Wasserstoff, einen gegebenenfalls substituierten $C_1$-$C_{10}$ Alkylrest oder einen hydrierten Arylrest;

X ein Brückenglied der Formeln $-CH_2-$, $-C(CH_3)_2-$, $-C(CF_3)_2-$, -CO-, - O-, -S-, $-SO_2-$,

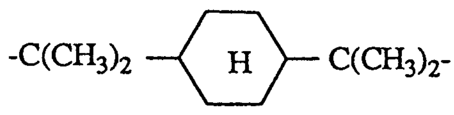 oder 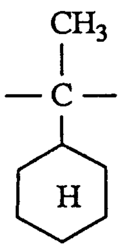 ;

Y = O (wenn n = 1) oder N (wenn n = 2);

m die Zahl 1, 2 oder 3;

n = 1 (wenn Y = O) oder 2 (wenn Y = N);

o die Zahl 1 bis 30; und

Z eine direkte Bindung oder ein Brückenglied der Formel $-CH_2-$ oder

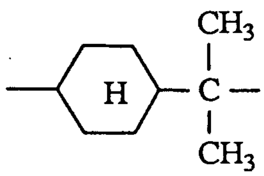 .

2. Cyclohexylgruppenhaltige Glycidylether der Formeln I bis IV gemäss Anspruch 1, dadurch gekennzeichnet, dass diese symmetrisch sind und X und Y in der Formel I in p-Stellung zueinander und die drei Y in der Formel II je in p-Stellung zu C beziehungsweise zu Z stehen.

3. Cyclohexylgruppenhaltige Glycidylether der Formeln I bis IV gemäss Anspruch 1, dadurch gekennzeichnet, dass

$R_1$, $R_2$ und $R_3$ Wasserstoff und/oder einen Alkylrest, vor allem einen $CH_3$-Rest; $R_4$ und $R_5$ Wasserstoff und/oder $CH_3$; $R_6$ Wasserstoff;

X ein Brückenglied der Formeln $-CH_2-$,$-C(CH_3)_2-$, $-C(CF_3)_2-$, -CO-, -O-oder $-SO_2-$;

Y O;

n die Zahl 1;

Z eine direkte Bindung oder das Brückenglied $-CH_2-$;

m die Zahl 2 oder 3; und o die Zahl 1 bis 20 und insbesondere 1 bis 15, bedeuten.

4. Cyclohexylgruppenhaltige Glycidylether der Formel I gemäss Anspruch 1, worin

$R_1$, $R_2$ und $R_3$ Wasserstoff und/oder einen $C_1$-$C_{10}$ Alkylrest, vorallem einen $CH_3$-Rest,

$R_4$ und $R_5$ Wasserstoff und/oder $CH_3$,

Y O,

n 1, und

X ein Brückenglied der Formeln $-CH_2-$, $-C(CH_3)_2-$, $-C(CF_3)_2-$, -CO-, - O- und $-SO_2$-bedeuten.

5. Cyclohexylgruppenhaltige Glycidylether der Formel II gemäss Anspruch 1, worin

$R_1$, $R_2$ und $R_3$ Wasserstoff und/oder einen $C_1$-$C_{10}$ Alkylrest, vorallem einen $CH_3$-Rest,

$R_4$ und $R_5$ Wasserstoff und/oder $CH_3$,

$R_6$ Wasserstoff,

Y O,
n 1, und
Z eine direkte Bindung oder ein Brückenglied $-CH_2-$ bedeuten.

**6.** Cyclohexylgruppenhaltige Glycidylether der Formel III gemäss Anspruch 1, worin

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
Y O,
n 1, und
m 2 oder 3 bedeuten.

**7.** Cyclohexylgruppenhaltige Glycidylether der Formel IV gemäss Anspruch 1, worin

$R_1$, $R_2$ und $R_3$ Wasserstoff und/oder einen $C_1$-$C_{10}$ Alkylrest, vorallem einen $CH_3$-Rest,
$R_4$ und $R_5$ Wasserstoff und/oder $CH_3$,
Y O,
n 1, und
o die Zahl 1 bis 20, vor allem 1 bis 15, bedeuten.

**8.** Verfahren zur Herstellung der cyclohexylgruppenhaltigen Glycidylether der Formeln I bis IV gemäss Anspruch 1, dadurch gekennzeichnet, dass

a) eine Verbindung der Formeln Ia, IIa, IIIa oder IVa

(Ia) 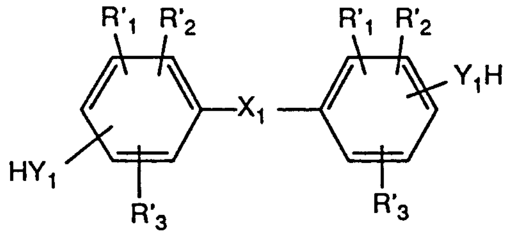 ,

(IIa) 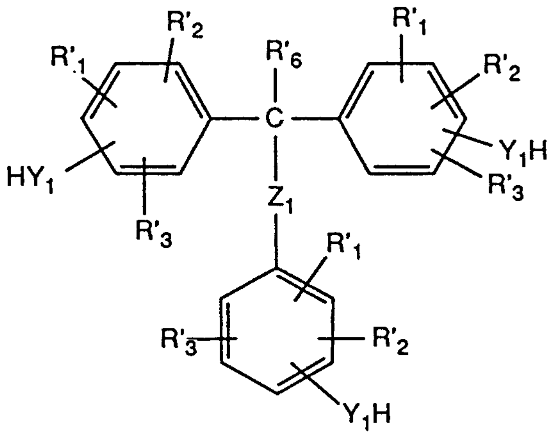 ,

(IIIa) 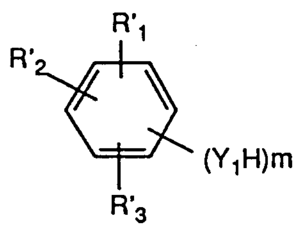 ,

(IVa) 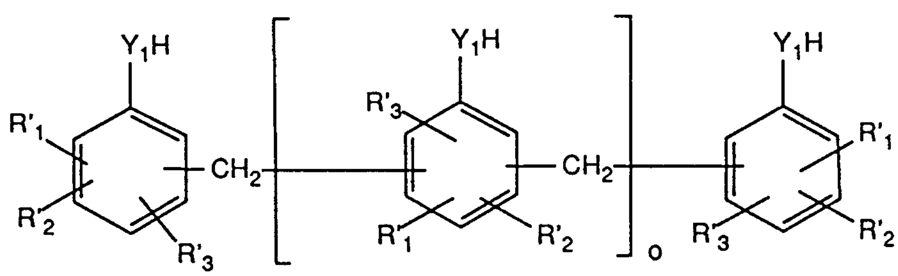 ,

mit Ethylenoxid oder dessen Derivaten der Formel

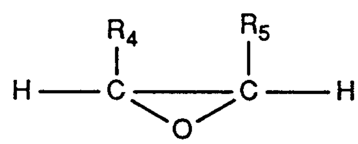

in Gegenwart einer Base in einem organischen Lösungsmittel bei einer Temperatur von 50 bis 200°C und einem Druck von 1 bis 10 bar umgesetzt wird,

b) die so erhaltenen Verbindungen der Formeln Ib, IIb, IIIb oder IVb

(Ib) 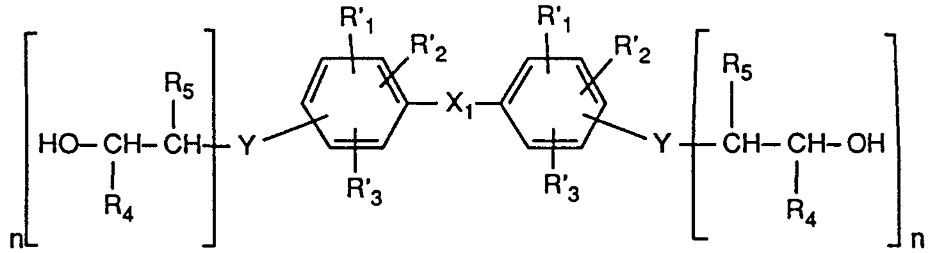 ,

(IIb) 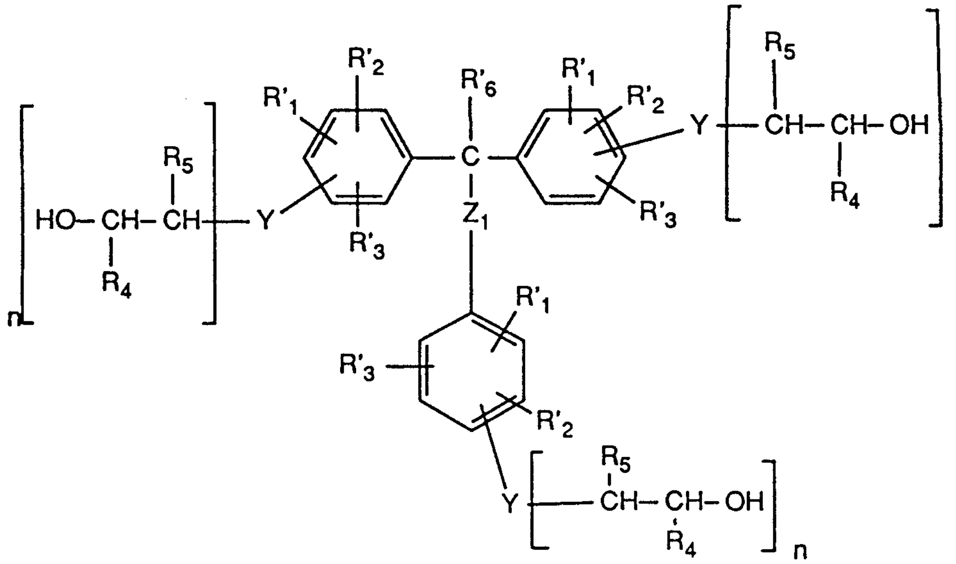 ,

(IIIb) 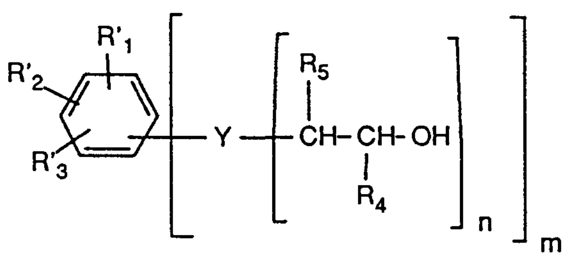 ,

(IVb)

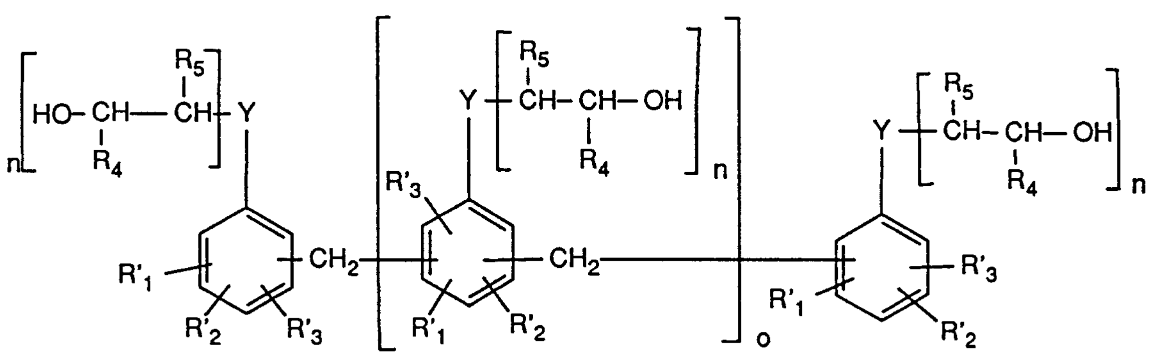 ,

mittels Wasserstoff und einem Katalysator zweckmässig in einem organischen Lösungsmittel bei einer Temperatur von 50 bis 200°C und einem Druck von 50 bis 200 bar hydriert werden, und

c) die so erhaltenen Verbindungen der Formeln Ic, IIc, IIIc oder IVc

(Ic) 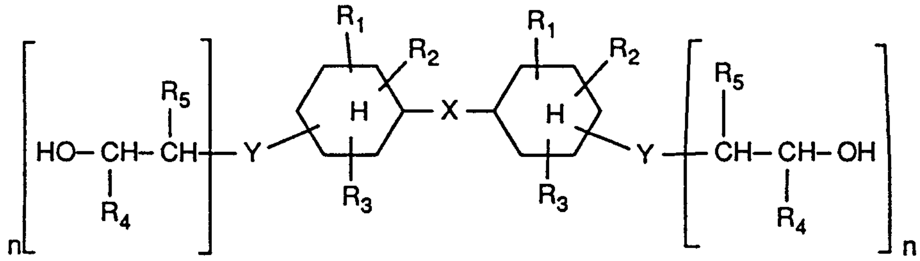 ,

(IIc)

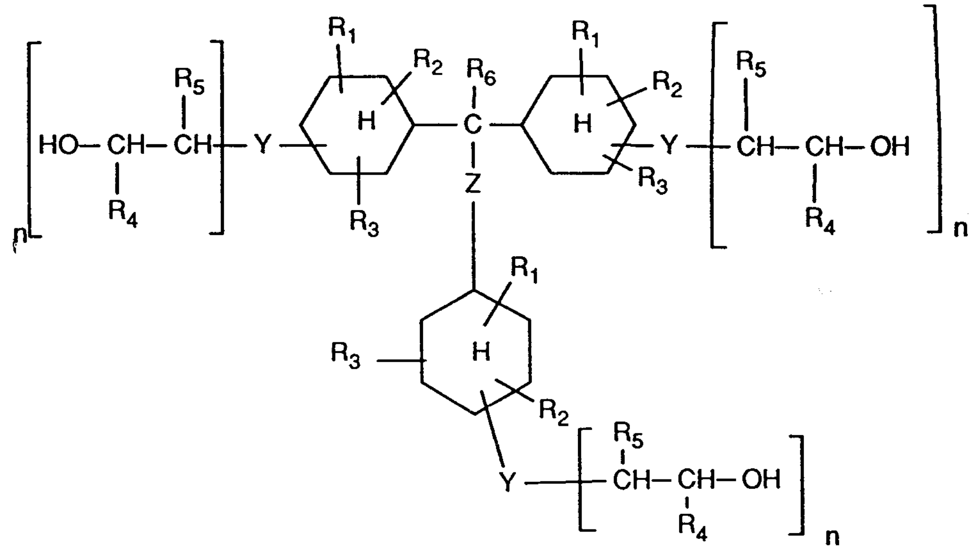

,

(IIIc)

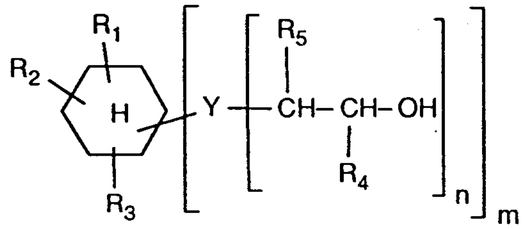

,

(IVc)

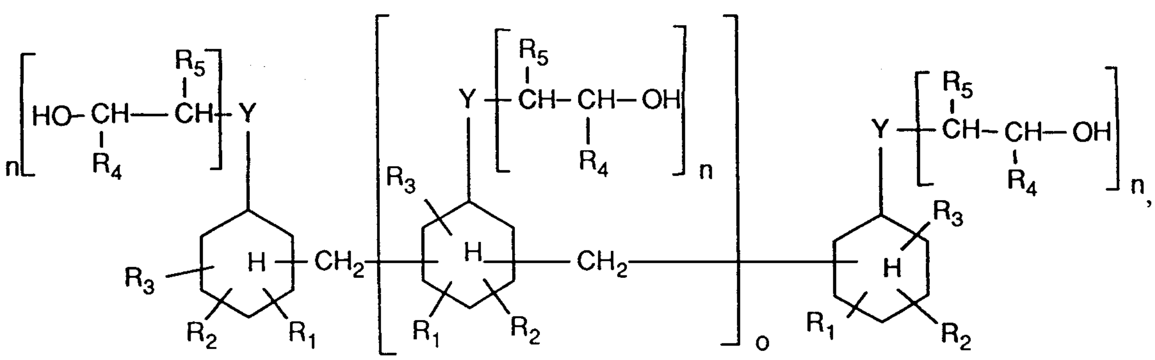

,

mit einem Epihalohydrin in Gegenwart eines Phasentransferkatalysators bei einer Temperatur von 40 bis 90°C und einem Druck von 6665 bis 26660 Pa (50 bis 200 Torr) unter Zugabe einer Lauge zu den Verbindungen der Formeln I, II, III beziehungsweise IV gemäss Anspruch 1 umgesetzt werden, in welchen Formeln Ia bis IVa, Ib bis IVb und Ic bis IVc; $R_1$ bis $R_6$, X, Y, Z, m, n und o die in Anspruch 1 angegebene Bedeutung haben.

$R'_1$, $R'_2$ und $R'_3$ unabhängig voneinander Wasserstoff, gegebenenfalls substituiertes $C_1$-$C_{10}$ Alkyl oder Aryl,
$R'_6$ Wasserstoff, gegebenenfalls substituiertes $C_1$-$C_{10}$ Alkyl oder Aryl,
$X_1$ ein Brückenglied der Formeln $-CH_2-$, $-C(CH_3)_2-$, $-C(CF_3)_2-$, -CO-, -O-, -S-, $-SO_2-$,

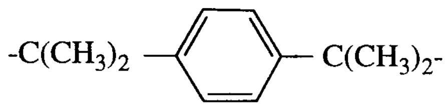 oder 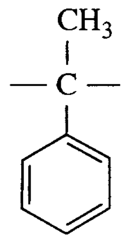 ,

$Y_1$ O oder NH, und $Z_1$ eine direkte Bindung oder ein Brückenglied der Formeln $-CH_2-$,

oder 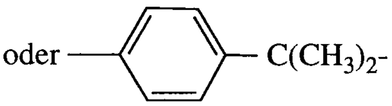

bedeuten.

**9.** Verfahren zur Herstellung der cyclohexylgruppenhaltigen Glycidylether der Formel I bis IV gemäss Anspruch 1, worin $R_4$ $CH_3$ und $R_5$ H bedeuten, dadurch gekennzeichnet, dass

a) die Verbindungen der Formeln $Ia_1$, $IIa_1$, $IIIa_1$ oder $IVa_1$

(Ia$_1$)

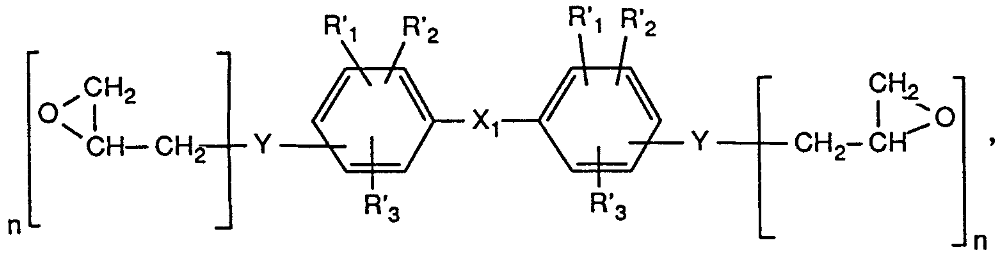

,

(IIa$_1$)

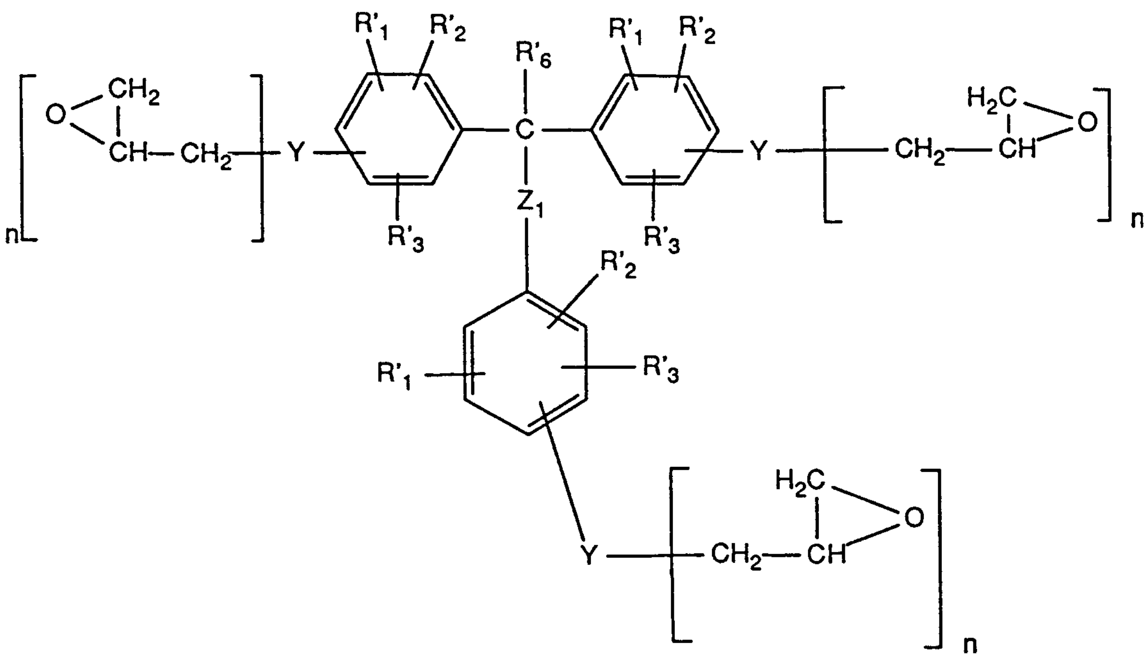

,

(IIIa$_1$)

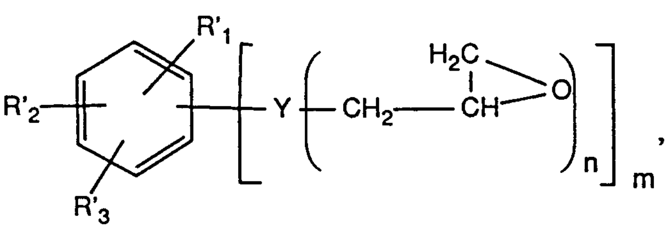

,

(IVa$_1$)

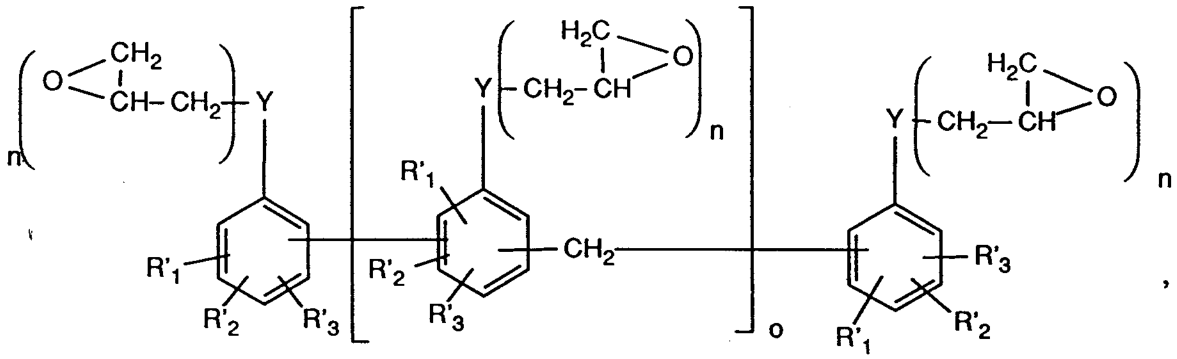

worin R'$_1$ bis R'$_3$, R'$_6$, X$_1$ und Z$_1$ die in Anspruch 8 angegebene Bedeutung haben, mittels Wasserstoff und einem Katalysator zweckmässig in einem organischen Lösungsmittel bei einer Temperatur von 50 bis 200°C und einem Druck von 50 bis 200 bar hydriert werden, und

b) die erhaltenen Verbindungen der Formeln Ib$_1$, IIb$_1$, IIIb$_1$ oder IVb$_1$

(Ib$_1$)

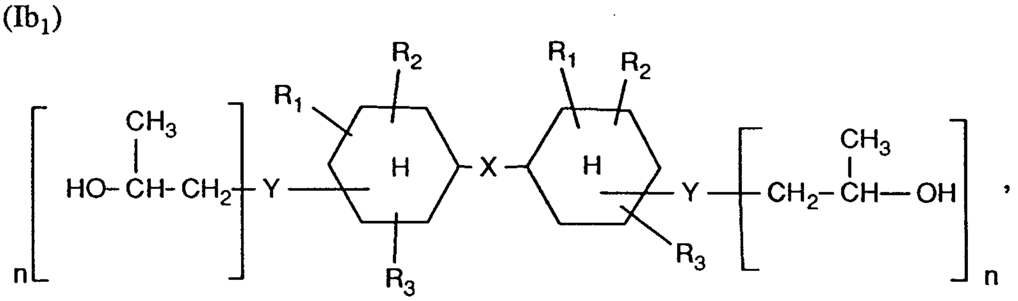

($IIb_1$)

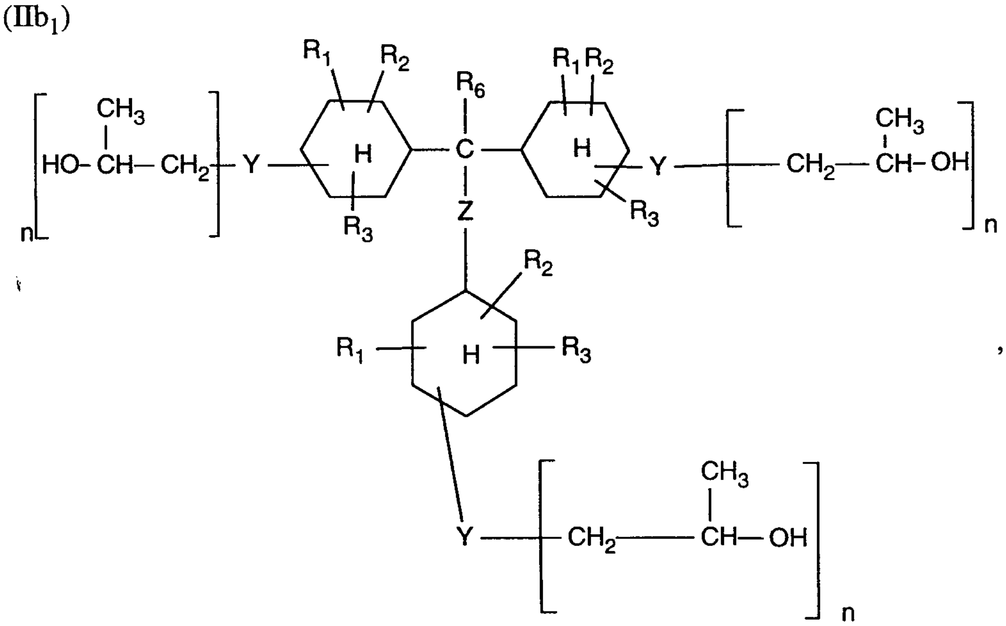

,

($IIIb_1$)

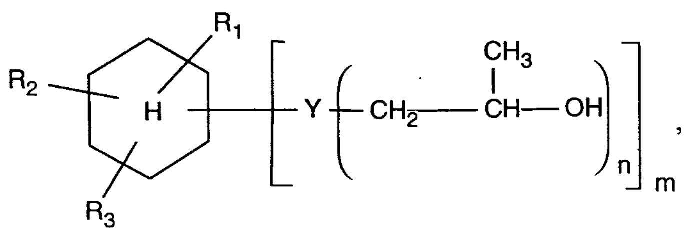

,

($IVb_1$)

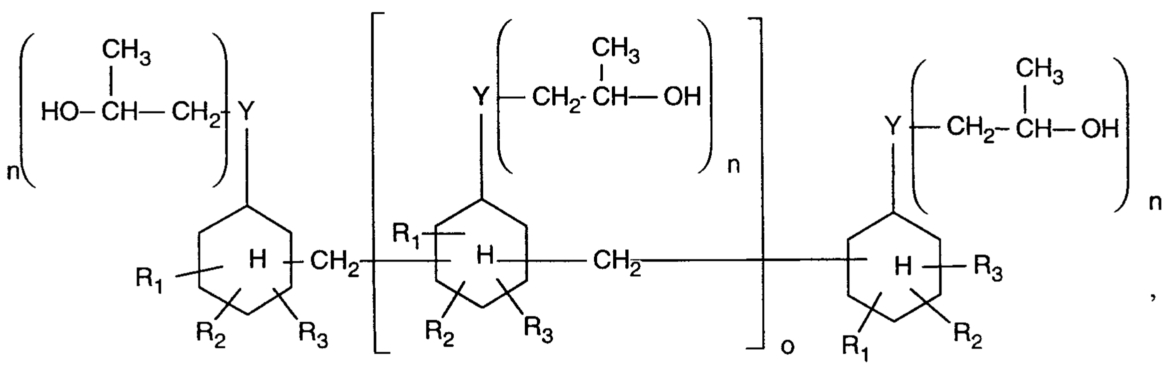

,

mit einem Epihalohydrin in Gegenwart eines Phasentransferkatalysators bei einer Temperatur von 40 bis 90°C und einem Druck von 6665 bis 26660 Pa (50 bis 200 Torr) unter Zugabe einer Lauge zu den Verbindungen der Formeln I, II, III beziehungsweise IV gemäss Anspruch 1 umgesetzt werden, in welchen Formeln $R_4$ für $CH_3$ und $R_5$ für H steht und $R_1$ bis $R_3$, $R_6$, X, Y, Z, m, n und o die in Anspruch 1 angegebene Bedeutung haben.

**10.** Verwendung der cyclohexylgruppenhaltigen Glycidylether der Formeln I bis IV gemäss dem Anspruch 1 oder den Ansprüchen 8 und 9 hergestellten cyclohexylgruppenhaltigen Glycidylether in Epoxidharz-Formulierungen als Raktivverdünner, Flexibilisatoren oder Adhäsionsverbesserern.

## Claims

1. A cyclohexyl-group-containing glycidyl ether of formula I, II, III or IV :

(I)

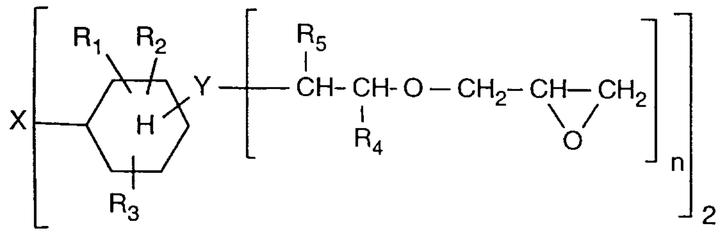

,

(II)

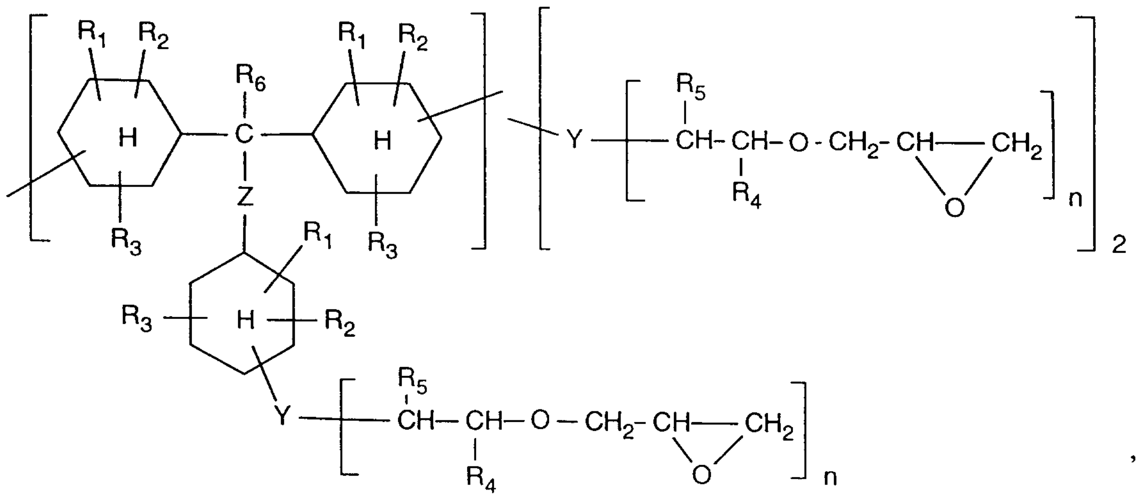

,

(III)

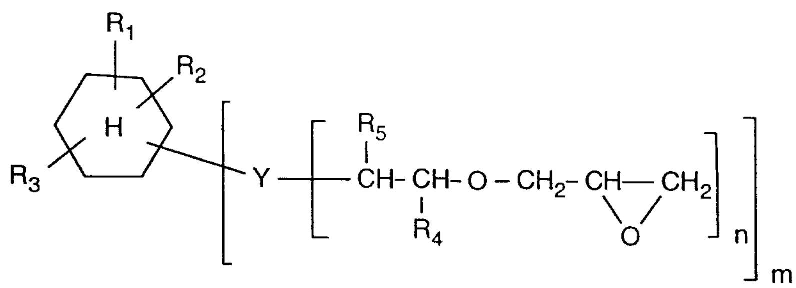

,

(IV)

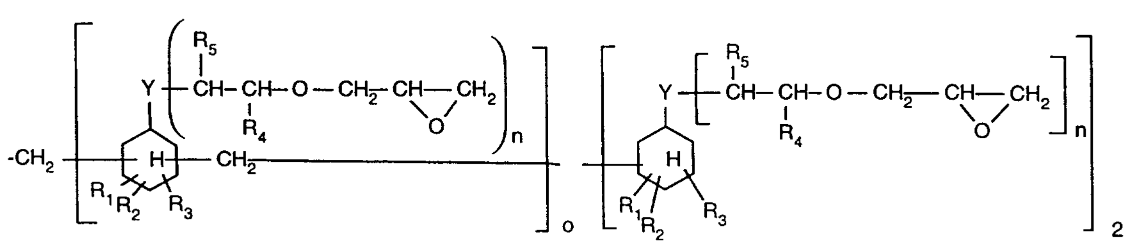

wherein the individual symbols are defined as follows:

$R_1$, $R_2$ and $R_3$ are each independently of the others hydrogen, an unsubstituted or substituted $C_1$-$C_{10}$alkyl radical or a hydrogenated aryl radical;
$R_4$ and $R_5$ are each independently of the other hydrogen or a $C_1$-$C_{20}$alkyl radical;
$R_6$ is hydrogen, an unsubstituted or substituted $C_1$-$C_{10}$alkyl radical or a hydrogenated aryl radical;
X is a bridge member of the formula $-CH_2-$, $-C(CH_3)_2-$, $-C(CF_3)_2-$, -CO-, -O-, -S-, $-SO_2-$,

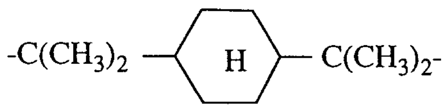 or 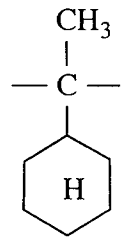 ;

Y = O (when n = 1) or N (when n = 2);
m is the number 1, 2 or 3;
n = 1 (when Y = O) or 2 (when Y = N);
o is a number from 1 to 30; and
Z is a direct bond or a bridge member of the formula $-CH_2-$ or

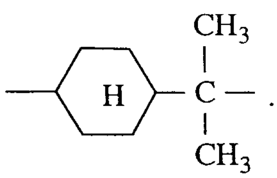

2. A cyclohexyl-group-containing glycidyl ether of formula I to IV according to claim 1, wherein the compound is symmetrical and X and Y in formula I are in the p-position relative to one another, and the three Ys in formula II are each in the p-position relative to C or Z, as the case may be.

3. A cyclohexyl-group-containing glycidyl ether of formula I to IV according to claim 1, wherein

$R_1$, $R_2$ and $R_3$ are hydrogen and/or an alkyl radical, especially a $CH_3$ radical;
$R_4$ and $R_5$ are hydrogen and/or $CH_3$;
$R_6$ is hydrogen;
X is a bridge member of the formula $-CH_2-$, $-C(CH_3)_2-$, $-C(CF_3)_2-$, -CO-, -O- or $-SO_2-$;
Y is O;
n is the number 1;
Z is a direct bond or the bridge member $-CH_2-$;
m is the number 2 or 3; and
o is a number from 1 to 20 and especially from 1 to 15.

4. A cyclohexyl-group-containing glycidyl ether of formula I according to claim 1, wherein

$R_1$, $R_2$ and $R_3$ are hydrogen and/or a $C_1$-$C_{10}$alkyl radical, especially a $CH_3$ radical,
$R_4$ and $R_5$ are hydrogen and/or $CH_3$,
Y is O,
n is 1, and
X is a bridge member of the formula $-CH_2-$, $-C(CH_3)_2-$, $-C(CF_3)_2-$, -CO-, -O- or $-SO_2-$.

5. A cyclohexyl-group-containing glycidyl ether of formula II according to claim 1, wherein

$R_1$, $R_2$ and $R_3$ are hydrogen and/or a $C_1$-$C_{10}$alkyl radical, especially a $CH_3$ radical,
$R_4$ and $R_5$ are hydrogen and/or $CH_3$,
$R_6$ is hydrogen,
Y is O,
n is 1, and
Z is a direct bond or a bridge member $-CH_2-$.

6. A cyclohexyl-group-containing glycidyl ether of formula III according to claim 1, wherein

$R_1$, $R_2$ and $R_3$ are hydrogen and/or a $C_1$-$C_{10}$alkyl radical, especially a $CH_3$ radical,
$R_4$ and $R_5$ are hydrogen and/or $CH_3$,
Y is O,
n is 1, and
m is 2 or 3.

7. A cyclohexyl-group-containing glycidyl ether of formula IV according to claim 1, wherein

$R_1$, $R_2$ and $R_3$ are hydrogen and/or a $C_1$-$C_{10}$alkyl radical, especially a $CH_3$ radical,
$R_4$ and $R_5$ are hydrogen and/or $CH_3$,
Y is O,
n is 1, and
o is a number from 1 to 20, especially from 1 to 15.

8. A process for the preparation of a cyclohexyl-group-containing glycidyl ether of formula I to IV according to claim 1, wherein

a) a compound of formula Ia, IIa, IIIa or IVa

(Ia) 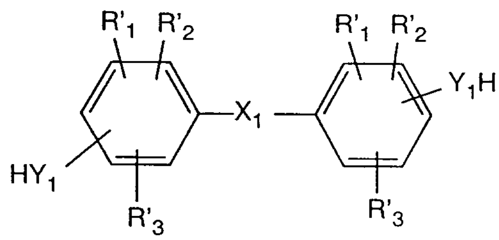 ,

(IIa) 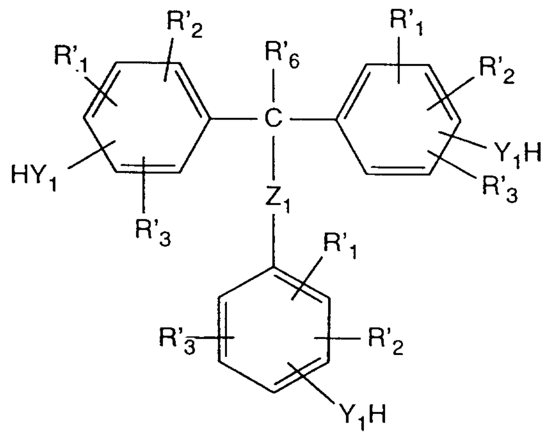 ,

(IIIa) 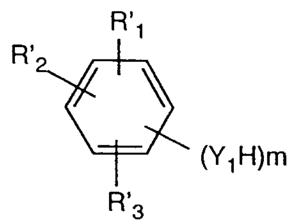 ,

(IVa) 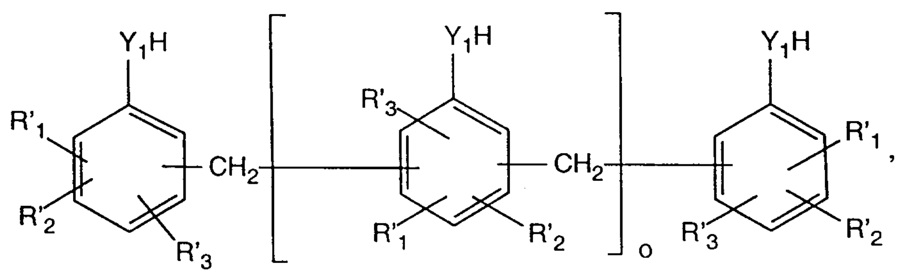

is reacted with ethylene oxide or a derivative thereof of the formula

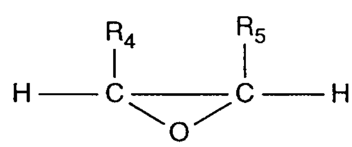

in the presence of a base in an organic solvent at a temperature of from 50 to 200°C and a pressure of from 1

to 10 bar,

b) a compound of formula Ib, IIb, IIIb or IVb so obtained

(Ib) 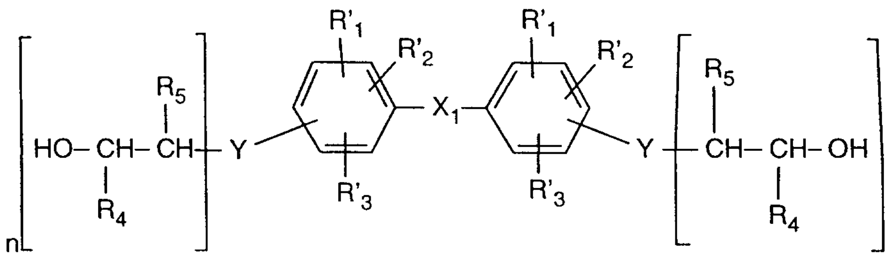 ,

(IIb) 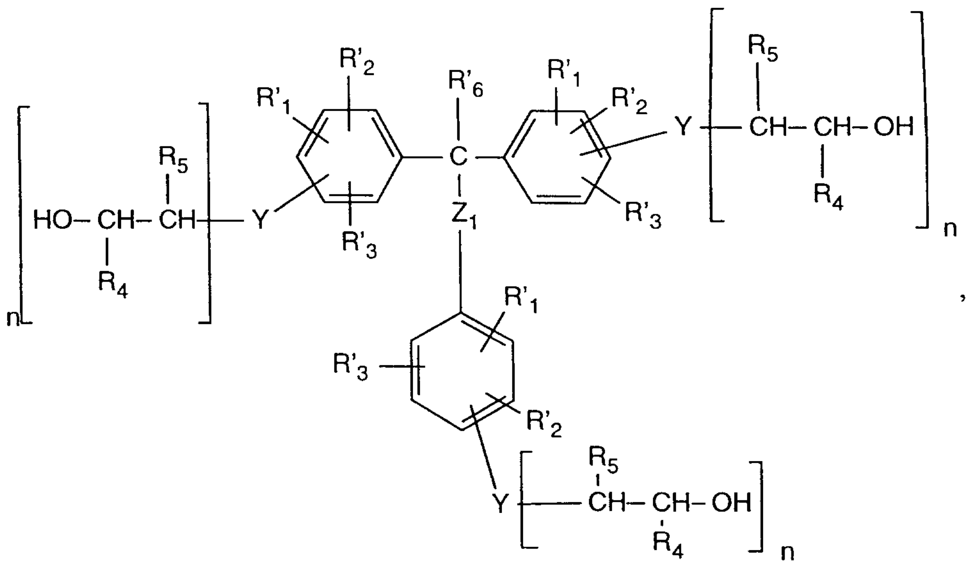 ,

(IIIb) 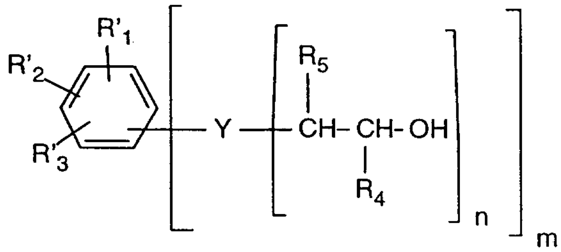 ,

(IVb)

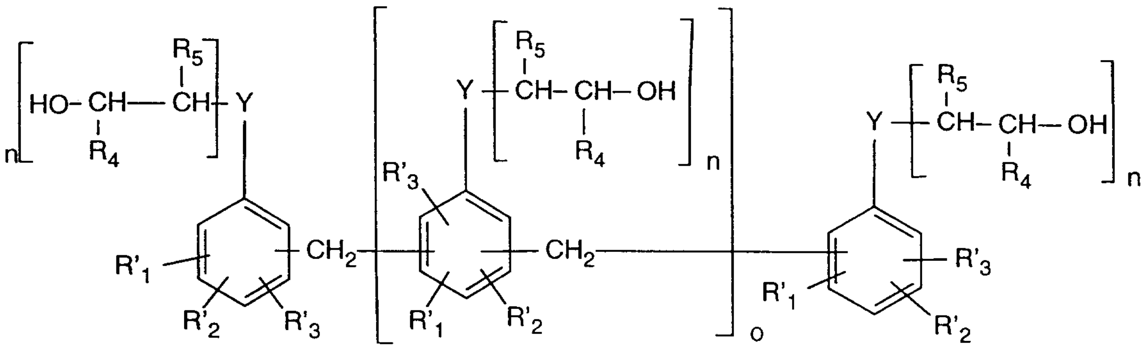 ,

is hydrogenated by means of hydrogen and a catalyst, advantageously in an organic solvent at a temperature of from 50 to 200°C and a pressure of from 50 to 200 bar, and

c) a compound of Ic, IIc, IIIc or IVc so obtained

(Ic) 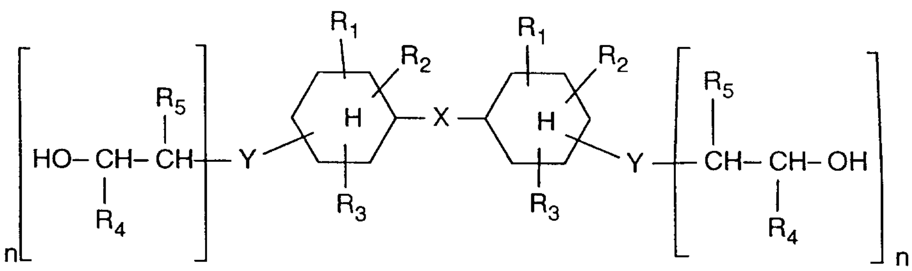 ,

(IIc)

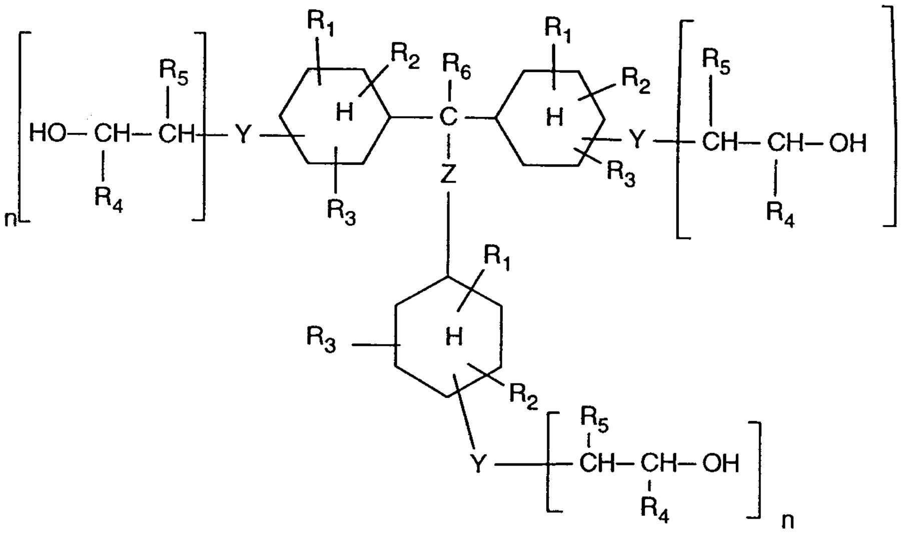

,

(IIIc)

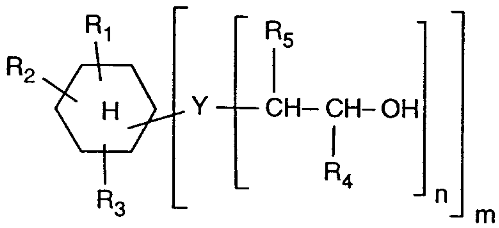

,

(IVc)

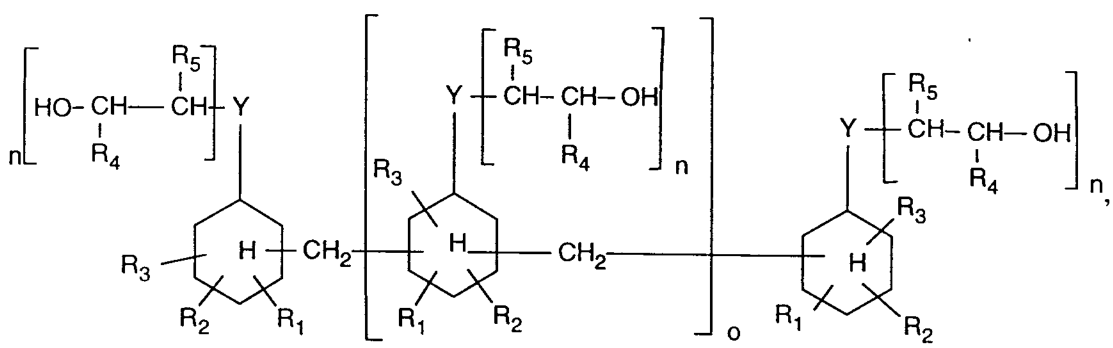

is reacted with an epihalohydrin in the presence of a phase transfer catalyst at a temperature of from 40 to 90°C and a pressure of from 6665 to 26 660 Pa (from 50 to 200 torr) with the addition of an alkaline solution to form a compound of formula I, II, III or IV, respectively, according to claim 1, in which formulae Ia to IVa, Ib to IVb and Ic to IVc $R_1$ to $R_6$, X, Y, Z, m, n and o are as defined in claim 1,

$R'_1$, $R'_2$ and $R'_3$ are each independently of the others hydrogen, unsubstituted or substituted $C_1$-$C_{10}$alkyl or aryl,
$R'_6$ is hydrogen, unsubstituted or substituted $C_1$-$C_{10}$alkyl or aryl,
$X_1$ is a bridge member of the formula $-CH_2-$, $-C(CH_3)_2-$, $-C(CF_3)_2-$, -CO-, -O-, -S-, $-SO_2-$,

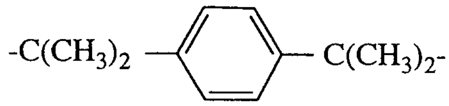 or 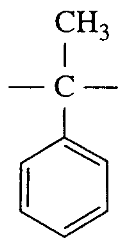 ,

$Y_1$ is O or NH, and
$Z_1$ is a direct bond or a bridge member of formula $-CH_2-$

or 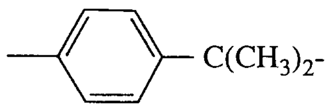 .

9. A process for the preparation of a cyclohexyl-group-containing glycidyl ether of formula I to IV according to claim 1 wherein $R_4$ is $CH_3$ and $R_5$ is H, wherein

a) a compound of formula $Ia_1$, $IIa_1$, $IIIa_1$ or $IVa_1$

($Ia_1$)

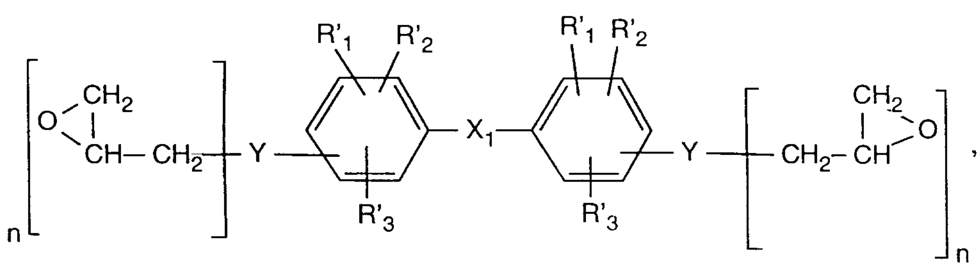 ,

(IIa$_1$)

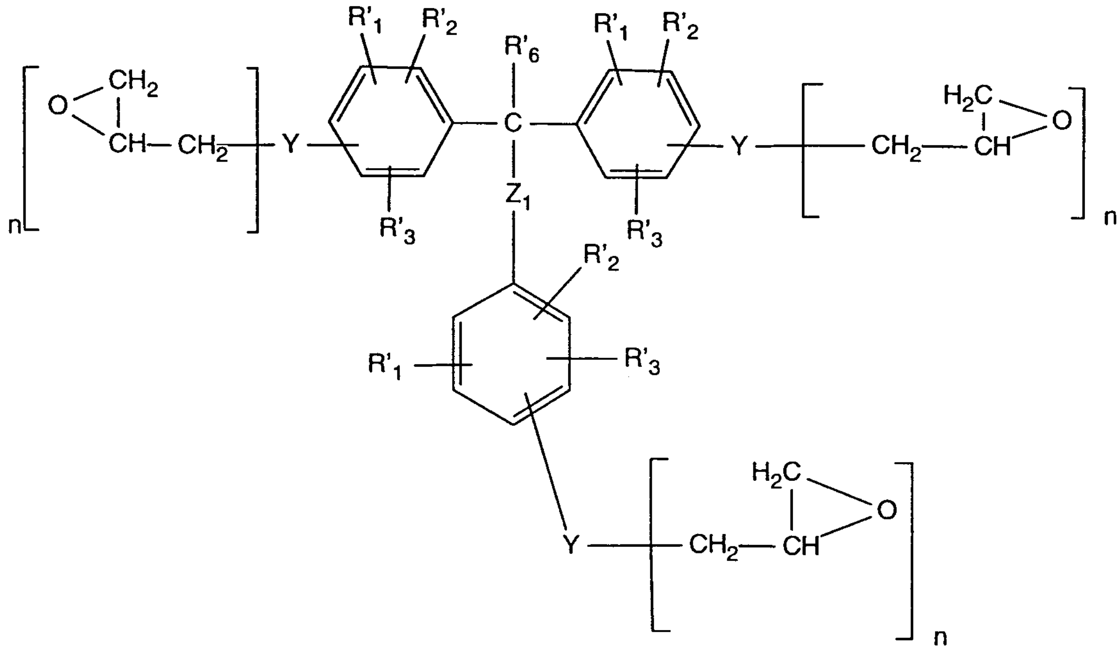

,

(IIIa$_1$)

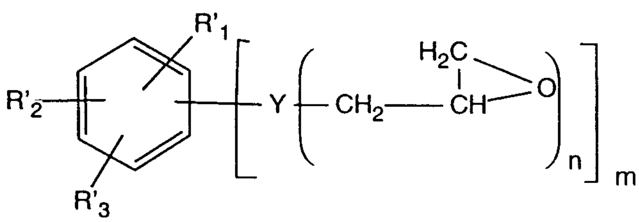

,

(IVa$_1$)

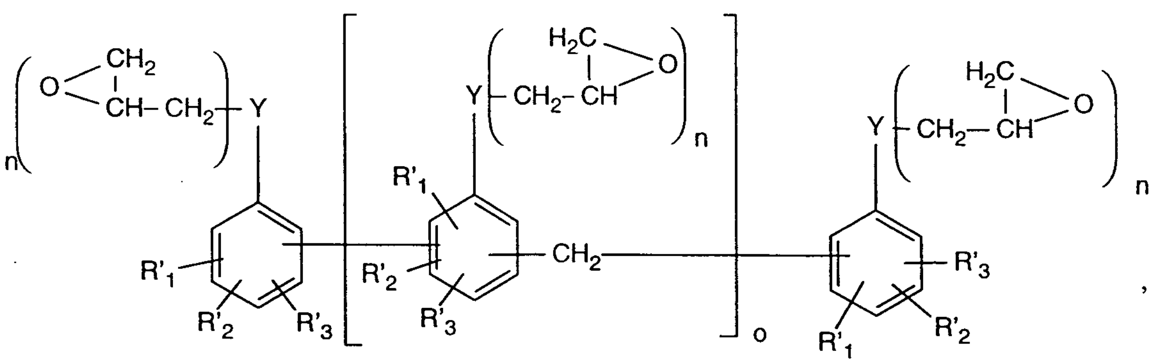

,

wherein R'$_1$ to R'$_3$, R'$_6$, X$_1$ and Z$_1$ are as defined in claim 8, is hydrogenated by means of hydrogen and a catalyst, advantageously in an organic solvent at a temperature of from 50 to 200°C and a pressure of from 50 to 200 bar, and

b) a resulting compound of formula Ib$_1$, IIb$_1$, IIIb, or IVb$_1$

(Ib$_1$)

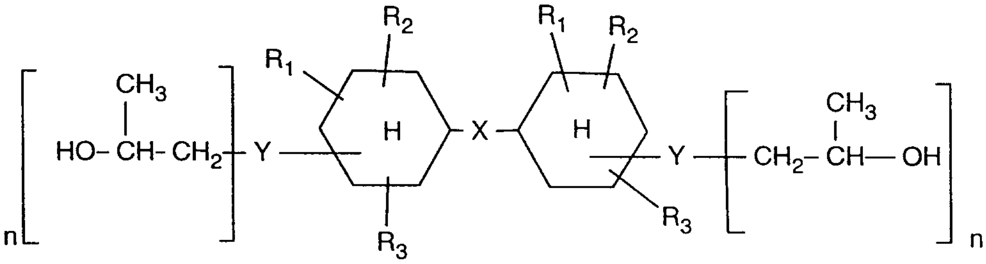

,

(IIb$_1$)

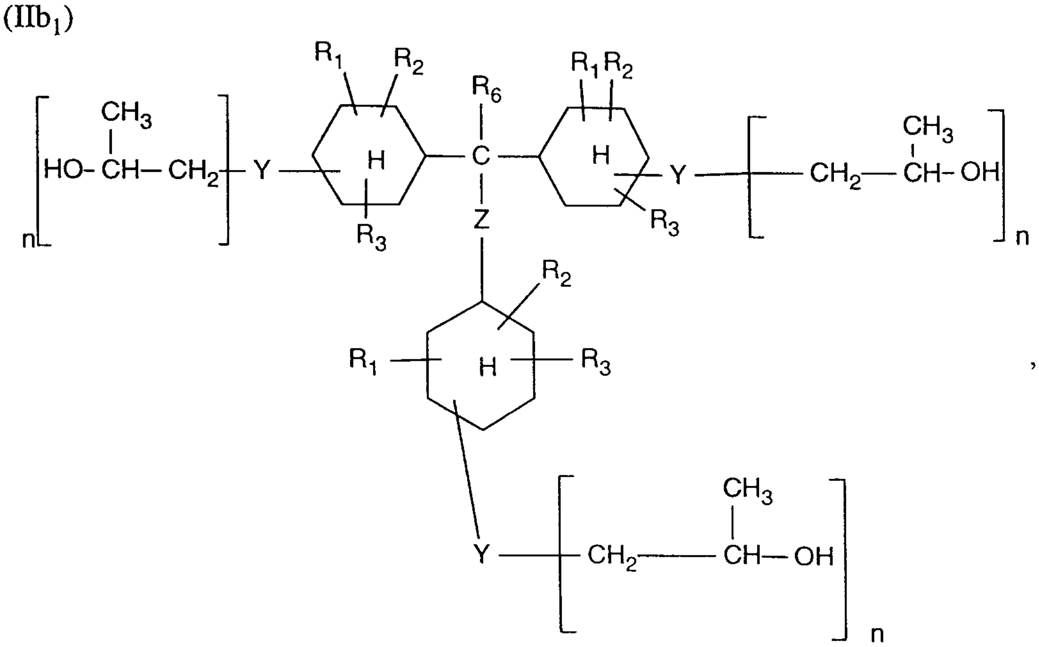

,

(IIIb$_1$)

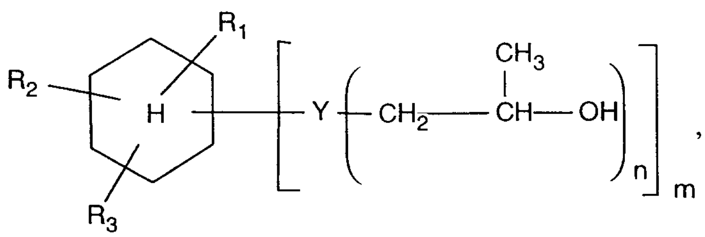

,

(IVb$_1$)

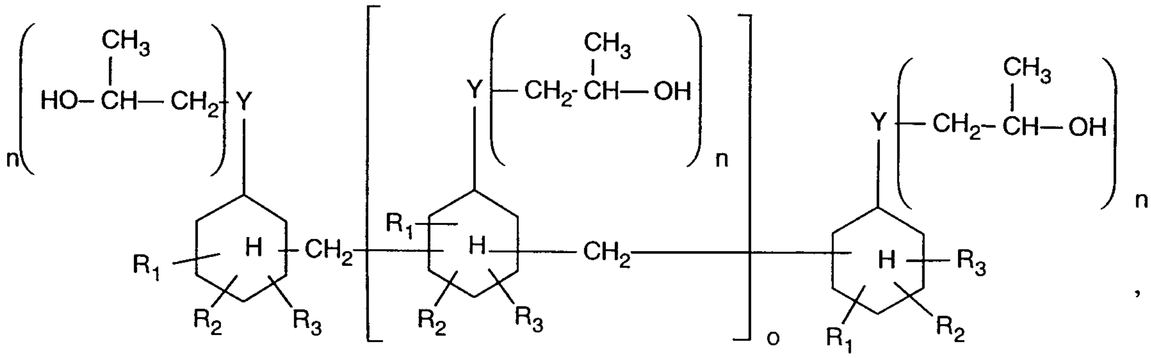

,

is reacted with an epihalohydrin in the presence of a phase transfer catalyst at a temperature of from 40 to 90°C and a pressure of from 6665 to 26 660 Pa (from 50 to 200 torr) with the addition of an alkaline solution to form a compound of formula I, II, III or IV, respectively, according to claim 1, in which formulae $R_4$ is $CH_3$ and $R_5$ is H and $R_1$ to $R_3$, $R_6$, X, Y, Z, m, n and o are as defined in claim 1.

**10.** The use of a cyclohexyl-group-containing glycidyl ether of formula I to IV according to claim 1 or of a cyclohexyl-group-containing glycidyl ether prepared according to claims 8 and 9 in epoxy resin formulations as a reactive diluent, flexibiliser or adhesion enhancer.

**Revendications**

1. Ethers glycidyliques contenant des groupes cyclohexyle de formules I, II, III, IV :

(I)

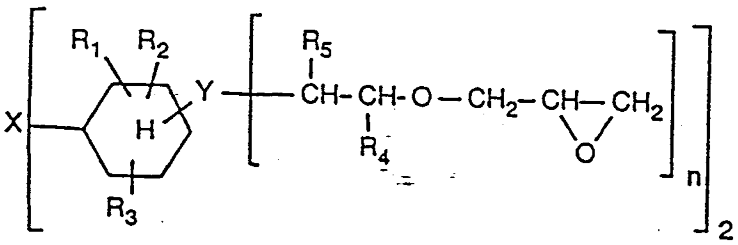

(II)

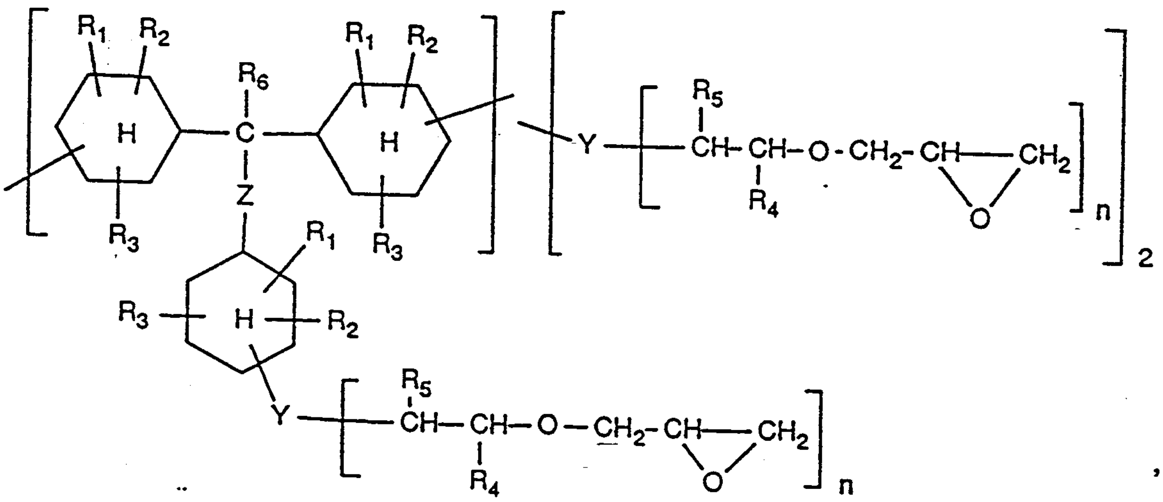

(III)

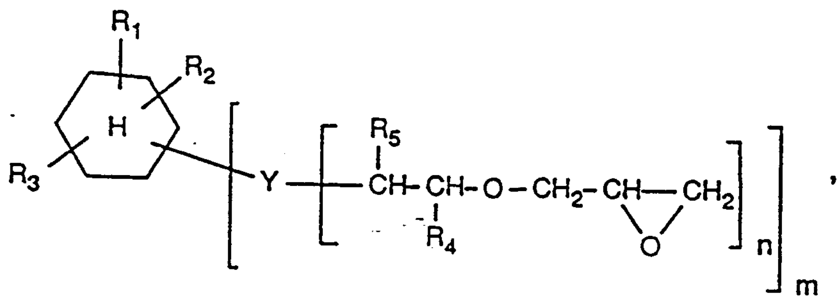

(IV)

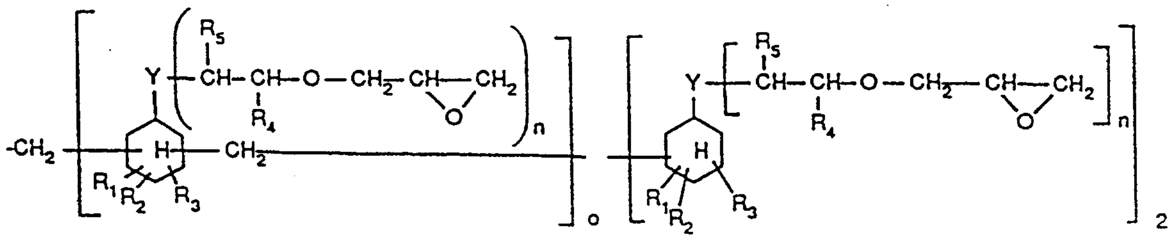

dans lesquelles les différents symboles ont les significations suivantes :

$R_1$, $R_2$ et $R_3$, indépendamment l'un de l'autre, représentent l'hydrogène, un reste alkyle en $C_1$-$C_{10}$ éventuellement substitué, ou un reste aryle hydrogéné ;

$R_4$ et $R_5$, indépendamment l'un de l'autre, représentent l'hydrogène ou un reste alkyle en $C_1$-$C_{20}$ ;
$R_6$ représente l'hydrogène, un reste alkyle en $C_1$-$C_{10}$ éventuellement substitué ou un reste aryle hydrogéné ;
X représente un élément de pont des formules $-CH_2-$, $-C(CH_3)_2-$, $-C(CF_3)_2-$, -CO-, -O-, -S-, $-SO_2-$ ;

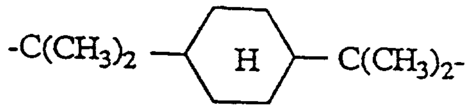 ou 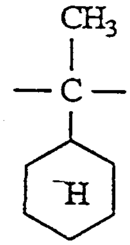 ;

Y = O (lorsque n = 1) ou N (lorsque n = 2) ;
m vaut 1, 2 ou 3 ;
n = 1 (lorsque Y = O) ou 2 (lorsque Y = N) ;
o va de 1 à 30 ; et
Z représente une liaison directe ou un élément de pont de formule $-CH_2-$ ou

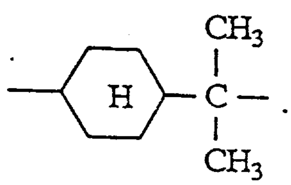

**2.** Ethers glycidyliques de formules I à IV contenant des groupes cyclohexyle selon la revendication 1, caractérisés en ce qu'ils sont symétriques et X et Y dans la formule I se trouvent en position para l'un par rapport à l'autre et les trois Y dans la formule II sont chacun en position para par rapport à C ou bien à Z.

**3.** Ethers glycidyliques de formules I à IV contenant des groupes cyclohexyle selon la revendication 1, caractérisés en ce que

$R_1$, $R_2$ et $R_3$ représentent l'hydrogène et/ou un reste alkyle, avant tout un reste $CH_3$ ; $R_4$ et $R_5$ représentent l'hydrogène et/ou $CH_3$ ; $R_6$ représente l'hydrogène ;
X est élément de pont des formules $-CH_2-$, $-C(CH_3)_2-$, $-C(CF_3)_2-$, -CO-, -O- ou $-SO_2-$ ;
Y représente O ;
n vaut 1 ;
Z est une liaison directe ou un élément de pont $-CH_2-$ ;
m vaut 2 ou 3 ; et o est un nombre de 1 à 20 et plus particulièrement de 1 à 15.

**4.** Ethers glycidyliques de formule I contenant des groupes cyclohexyle selon la revendication 1, où

$R_1$, $R_2$ et $R_3$ représentent l'hydrogène et/ou un reste alkyle en $C_1$-$C_{10}$, avant tout un reste $CH_3$,
$R_4$ et $R_5$ représentent l'hydrogène et/ou $CH_3$,
Y représente O,
n vaut 1, et
X représente un élément de pont de formule $-CH_2-$, $-C(CH_3)_2-$, $-C(CF_3)_2-$, -O- et $SO_2-$.

**5.** Ethers glycidyliques de formule II contenant des groupes cyclohexyle selon la revendication 1, où

$R_1$, $R_2$ et $R_3$ représentent l'hydrogène et/ou un reste alkyle en $C_1$-$C_{10}$, avant tout un reste $CH_3$,
$R_4$ et $R_5$ représentent l'hydrogène et/ou $CH_3$,
Y représente O,
n vaut 1, et
Z représente une liaison directe ou un élément de pont $-CH_2-$.

**6.** Ethers glycidyliques de formule III contenant des groupes cyclohexyle selon la revendication 1, où

$R_1$, $R_2$ et $R_3$ représentent l'hydrogène et/ou un reste alkyle en $C_1$-$C_{10}$, avant tout un reste $CH_3$,
$R_4$ et $R_5$ représentent l'hydrogène et/ou $CH_3$,
Y représente O,
n vaut 1, et
m vaut 2 ou 3.

**7.** Ethers glycidyliques de formule IV contenant des groupes cyclohexyle selon la revendication 1, où

$R_1$, $R_2$ et $R_3$ représentent l'hydrogène et/ou un reste alkyle en $C_1$-$C_{10}$, avant tout un reste $CH_3$,
$R_4$ et $R_5$ représentent l'hydrogène et/ou $CH_3$,
Y représente O,
n vaut 1, et
o va de 1 à 20, avant tout de 1 à 15.

**8.** Procédé de préparation des éthers glycidyliques de formules I à IV contenant des groupes cyclohexyle selon la revendication 1, caractérisé en ce que

a) on fait réagir un composé de formules Ia, IIa, IIIa ou IVa

(Ia) 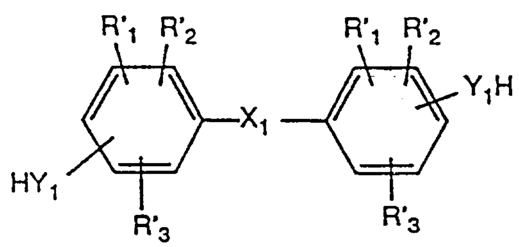 ,

(IIa) 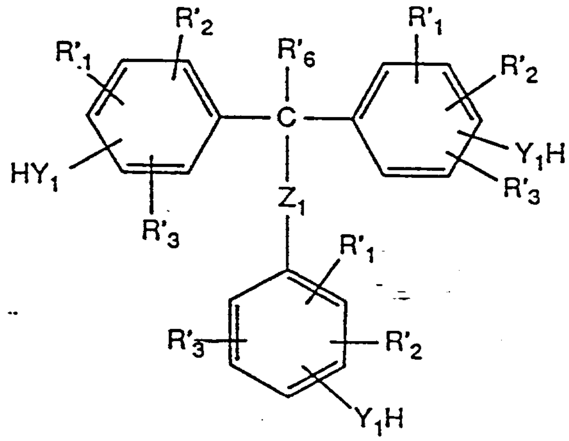 ,

(IIIa) 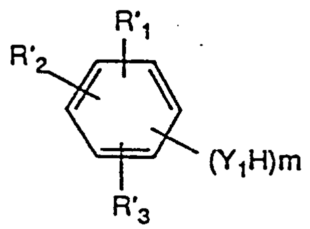 ,

(IVa) 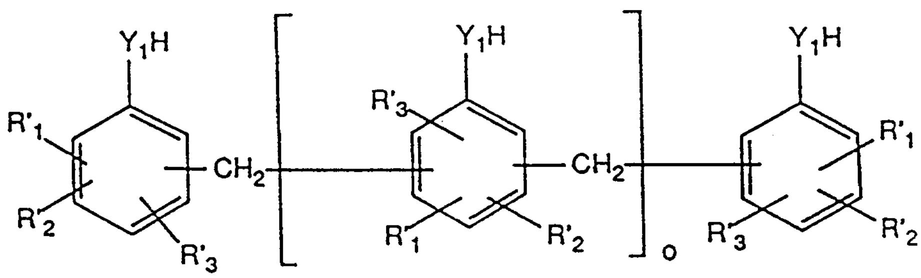 ,

avec l'oxyde d'éthylène ou ses dérivés de formule

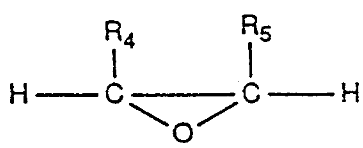

en présence d'une base, dans un solvant organique, à une température de 50 à 200 °C et une pression de 1 à 10 bars,

b) on hydrogène les composés de formules Ib, IIb, IIIb et IVb ainsi obtenus

(Ib) 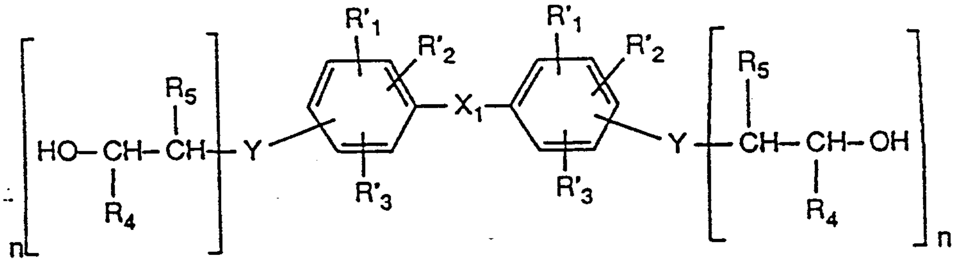 ,

(IIb) 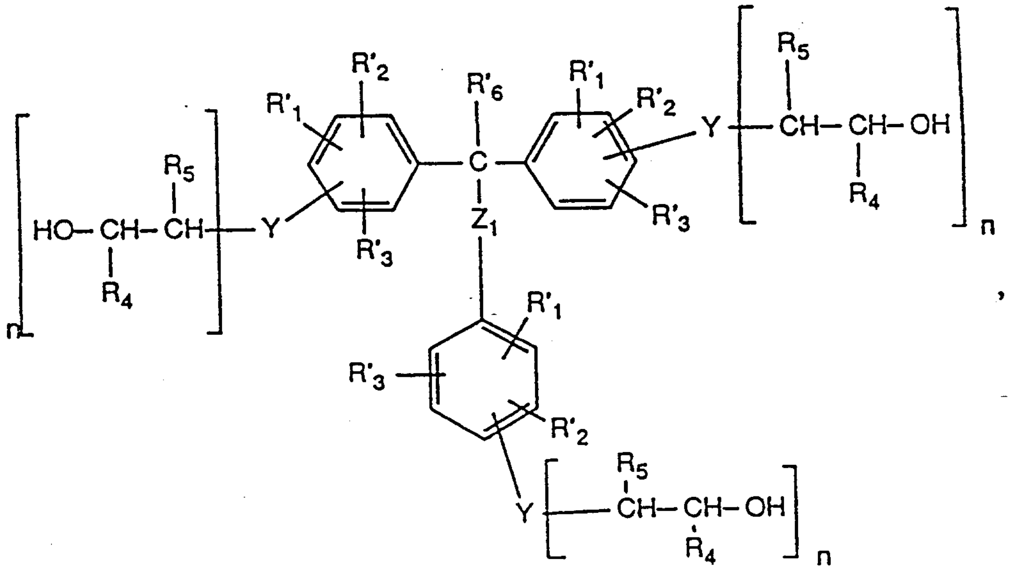 ,

(IIIb) 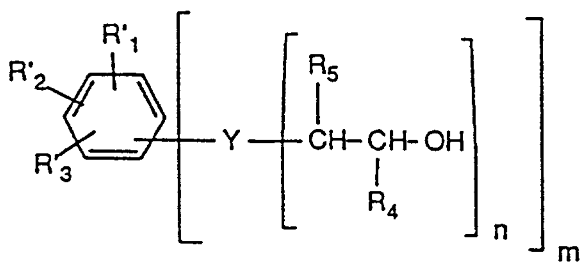 ,

(IVb)

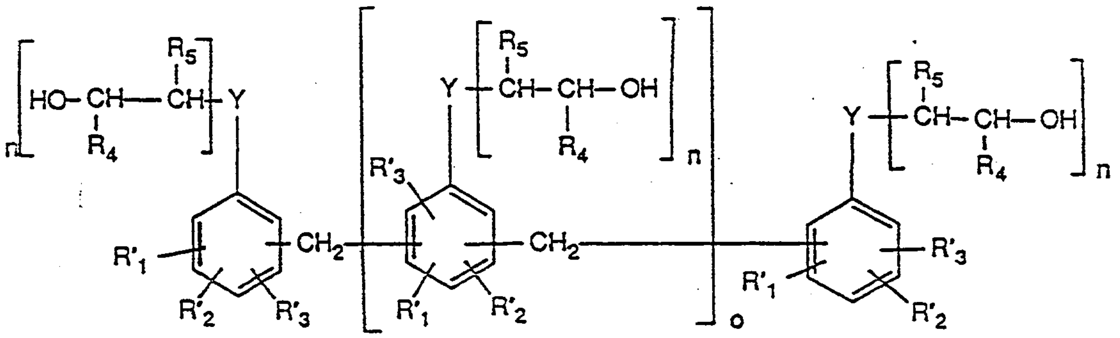

par l'hydrogène et un catalyseur, de façon appropriée dans un solvant organique, à une température de 50 à 200 °C et une pression de 50 à 200 bars, et,

c) on fait réagir les composés ainsi obtenus de formules Ic, IIc, IIIc ou IVc

(Ic)

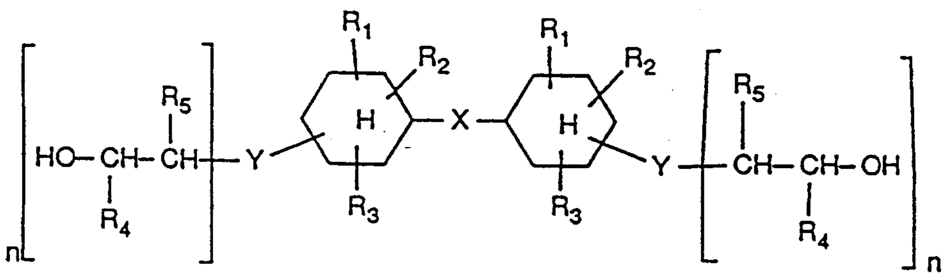

,

(IIc)

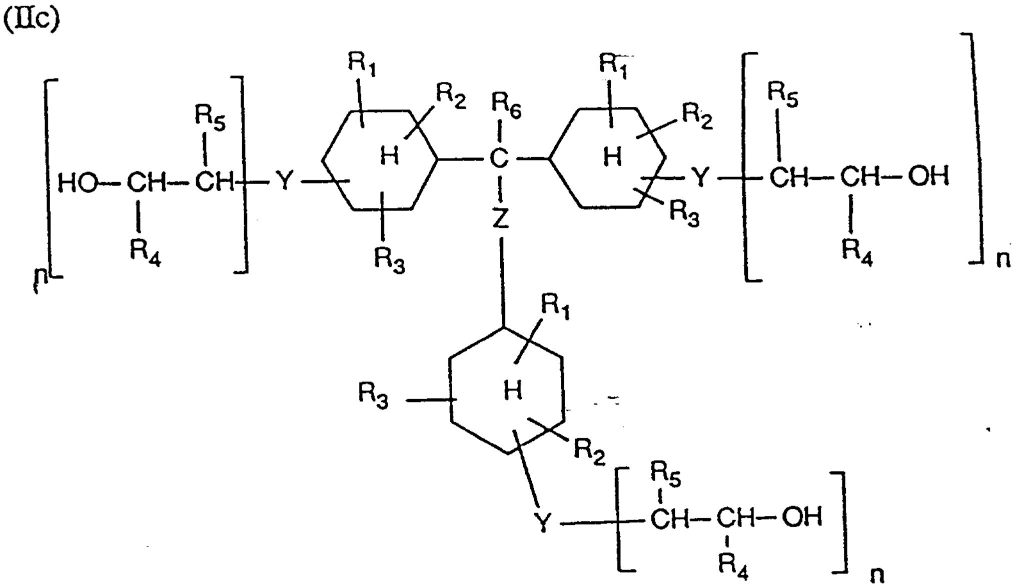

,

(IIIc) 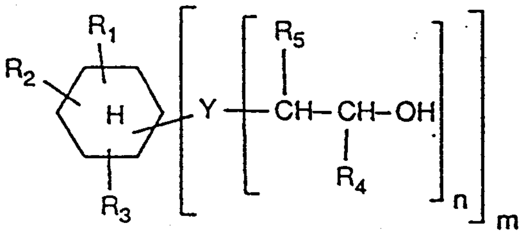 ,

(IVc)

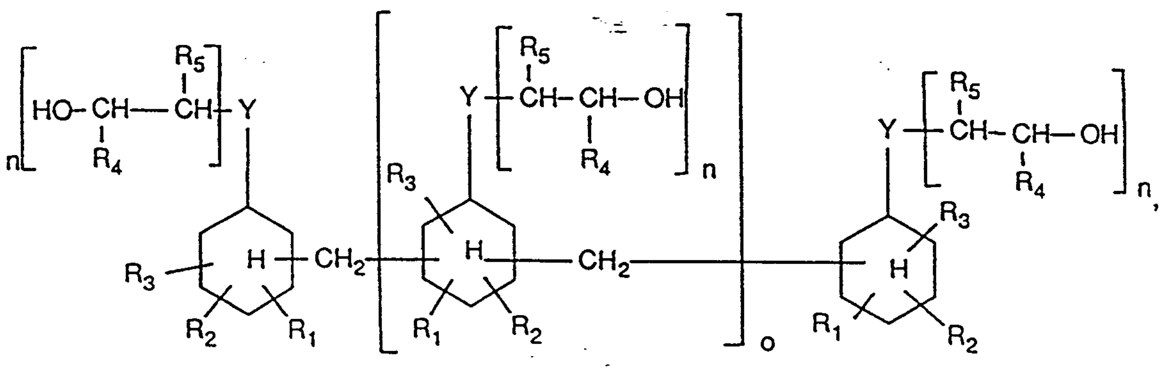 ,

avec une épihalohydrine en présence d'un catalyseur de transfert de phases à une température de 40 à 90 °C et une pression de 6 665 à 26 660 Pa (de 50 à 200 torrs), en ajoutant une lessive au composés de formules I, II, III ou IV selon la revendication 1, dans lesquelles formules Ia à IVa, Ib à IVb et Ic à IVc, $R_1$ à $R_6$, X, Y, Z, m, n et o ont les significations données dans la revendication 1.

$R_1'$, $R_2'$ et $R_3'$, indépendamment l'un de l'autre, représentent l'hydrogène, alkyle en $C_1$-$C_{10}$ ou aryle éventuellement substitué,
$R_6'$ représente l'hydrogène, aryle ou alkyle en $C_1$-$C_{10}$ éventuellement substitué,
$X_1$ représente un élément de pont de formules $-CH_2-$, $-C(CH_3)_2-$, $-C(CF_3)_2-$, -CO-, -O-, -S-, $-SO_2-$,

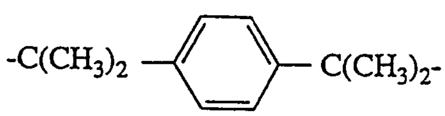 ou 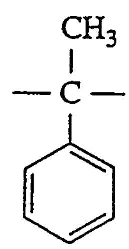 ,

$Y_1$ représente O ou NH et $Z_1$ une liaison directe ou un élément de pont de formule $-CH_2-$ ou

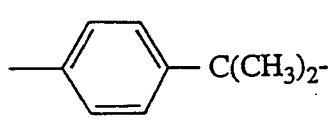

**9.** Procédé pour la préparation des éthers glycidyliques contenant des groupes cyclohexyle de formules I à IV selon la revendication 1, où $R_4$ représente $CH_3$ et $R_5$ représente H, caractérisé en ce que

a) on hydrogène les composés de formules $Ia_1$, $IIa_1$, $IIIa_1$ ou $IVa_1$

($Ia_1$)

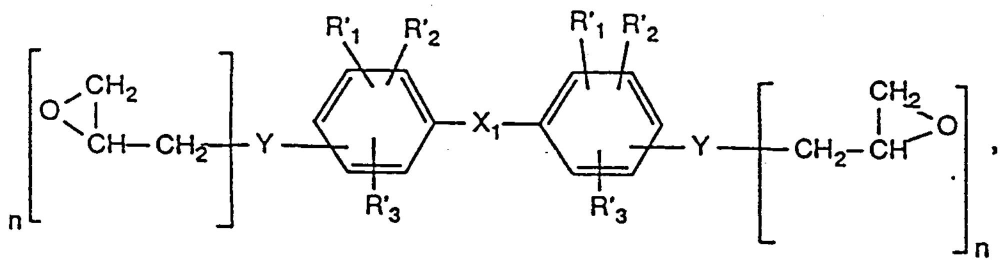

,

($IIa_1$)

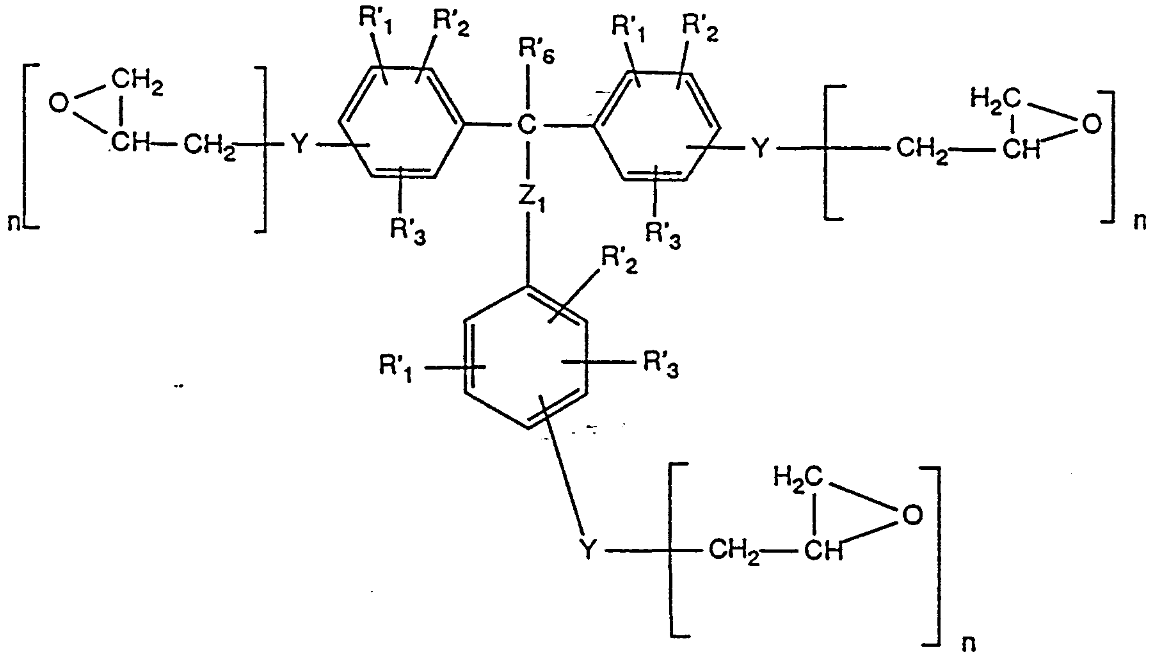

,

($IIIa_1$)

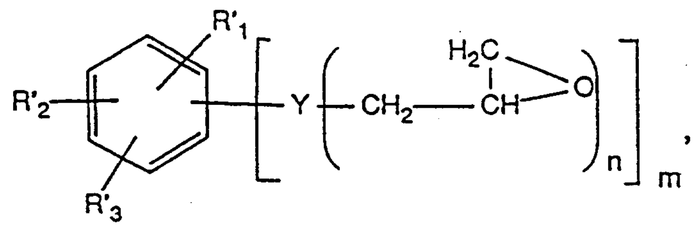

,

($IVa_1$)

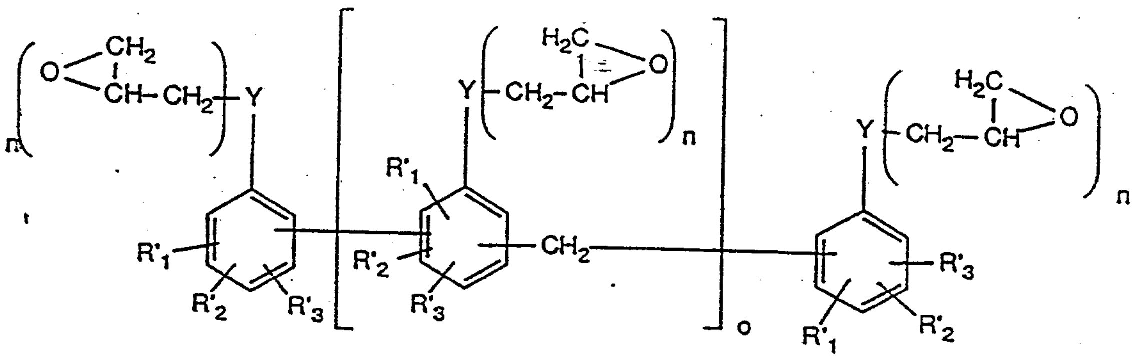

dans lesquelles $R'_1$ à $R'_3$, $R'_6$, $X_1$ et $Z_1$ ont la signification donnée dans la revendication 8, par l'hydrogène et uncatalyseur, de façon appropriée dans un solvant organique, à une température de 50 à 200 °C et une pression de 50 à 200 bars, et

b) on fait réagir les composés obtenus de formules $Ib_1$, $IIb_1$, $IIIb_1$ ou $IVb_1$

($Ib_1$)

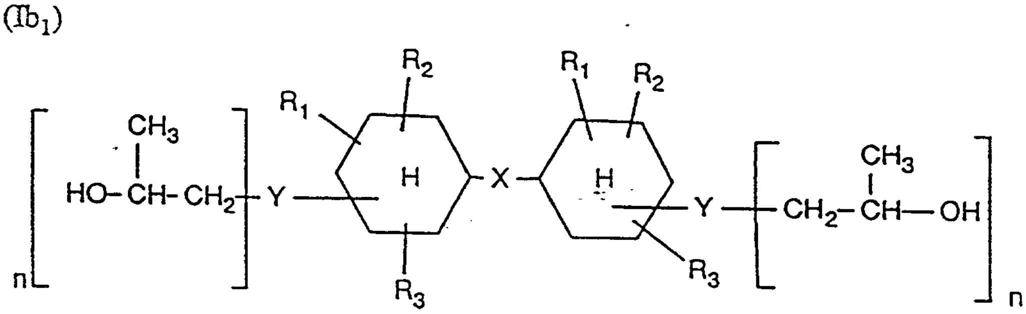

,

(IIb$_1$)

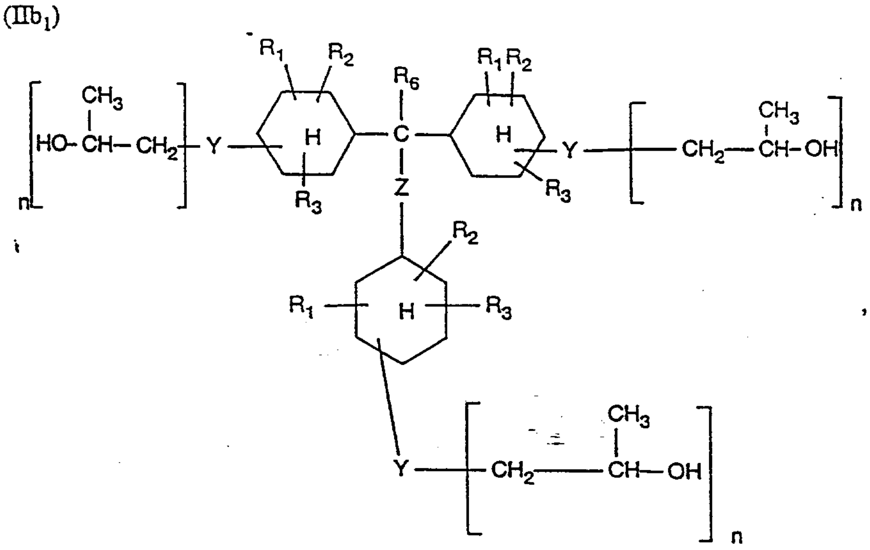

,

(IIIb$_1$)

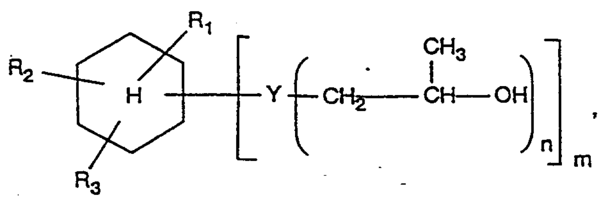

,

(IVb$_1$)

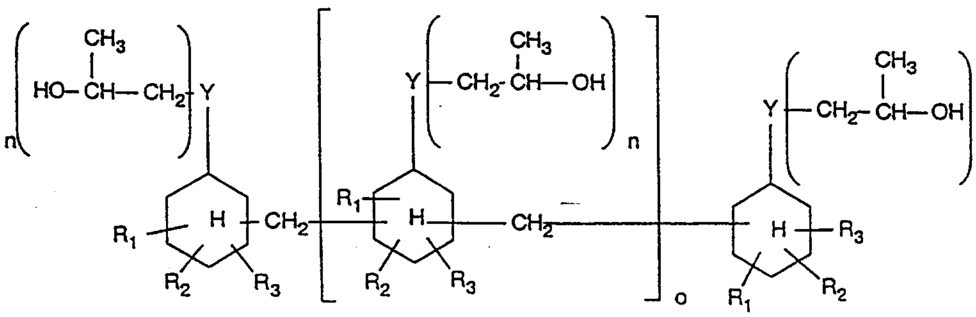

,

avec une épihalohydrine en présence d'un catalyseur de transfert de phases, à une température de 40 à 90 °C et une pression de 6 665 à 26 660 Pa (de 50 à 200 torrs), en ajoutant une lessive aux composés de formules I, II, III ou IV selon la revendication 1, dans lesquelles formules $R_4$ représente $CH_3$ et $R_5$ représente H et $R_1$ à $R_3$, $R_6$, X, Y, Z, m, n et o ont les significations données dans la revendication 1.

**10.** Utilisation des éthers glycidyliques contenant des groupes cyclohexyle de formules I à IV selon la revendication 1: où des éthers glycidyliques contenant des groupes cyclohexyle préparés selon les revendications 8 et 9, dans les formulations de résines époxydes comme diluants réactifs, plastifiants ou agents améliorant l'adhésion.